(19) Europäisches Patentamt
European Patent Office
Office européen des brevets

(11) Numéro de publication: **0 213 006**
**B1**

(12) **FASCICULE DE BREVET EUROPÉEN**

(45) Date de publication du fascicule du brevet: **25.01.89**

(21) Numéro de dépôt: **86401513.6**

(22) Date de dépôt: **08.07.86**

(51) Int. Cl.⁴: **C 07 D 307/81**, C 07 D 307/83, C 07 D 405/06, C 07 D 495/04, A 61 K 31/34, A 61 K 31/47 // (C07D495/04, 333:00, 221:00)

(54) **Nouveaux dérivés du dihydro-2,3 benzofuranne, leurs procédés de préparation et les compositions pharmaceutiques qui les contiennent.**

(30) Priorité: 08.07.85 FR 8510459

(43) Date de publication de la demande: **04.03.87 Bulletin 87/10**

(45) Mention de la délivrance du brevet: **25.01.89 Bulletin 89/4**

(84) Etats contractants désignés: **AT BE CH DE FR GB IT LI LU NL SE**

(56) Documents cité:
**DK-A-845 830**
**US-A-3 156 688**

**JOURNAL OF PHARMACEUTICAL SCIENCES, vol. 74, no. 1, janvier 1985, pages 44-46, American Pharmaceutical Association, US; J.J. TEGELER et al.: "Efforts toward combined analgesic/antidepressants: synthesis and evaluation of (3-aryl-2,3-dihydrobenzofuran-3-yl)alkanamines"**

**Le dossier contient des informations techniques présentées postérieurement au dépôt de la demande et ne figurant pas dans le présent fascicule.**

(73) Titulaire: **ADIR ET COMPAGNIE, 22, rue Garnier, F-92200 Neuilly- sur- Seine (FR)**

(72) Inventeur: **Lavielle, Gilbert, 1 avenue Lily, F-78170 La Celle Saint- Cloud (FR)**
Inventeur: **Gargouil, Yves- Michel, 38 rue Michel Ange, F-75016 Paris (FR)**
Inventeur: **Vilaine, Jean- Paul, 5 rue Arthur Ranc, F-92350 Le Plessis Robinson (FR)**

**EP 0 213 006 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne de nouveaux dérivés du dihydro-2,3 benzofuranne, leurs procédés de préparation et les compositions pharmaceutiques les renfermant.

On connaît de nombreux dérivés benzofuranniques possédant d'intéressantes propriétés pharmacologiques, notamment l'amiodarone et la benziodarone, utilisés dans le traitement de l'angine de poitrine. D'une façon plus surprenante, la littérature mentionne peu de dérivés du dihydro-2,3 benzofuranne pharmacologiquement actifs. En effet, certains aminoalkyl-3 dihydro-2,3 benzofurannes, non substituées par des radicaux méthoxy, ont été décrits par Tegeler J. et coll. dans J. Pharm. Sc. (1985), 74, N° 1,44-46 et dans DK-A-845 830; et ces composés possèdent une activité antidépressive en général modeste. D'autres aminométhyl-3 dihydro-2,3 benzofurannes (brevet US N° 3 156 688) ont montré chez le chat une activité hypotensive à des doses très importantes (25 mg/kg par voie intraveineuse).

A l'inverse des composés de l'état de l'art, les composés de la présente invention et en particulier ceux qui comportent un ou plusieurs radicaux alcoxy, ont montré de très intéressantes propriétés pharmacologiques, notamment comme modulateurs des mouvements transmembranaires et intracellulaires du calcium.

La présente invention a plus particulièrement pour objet les dérivés du dihydro-2,3 benzofuranne, de formule générale I:

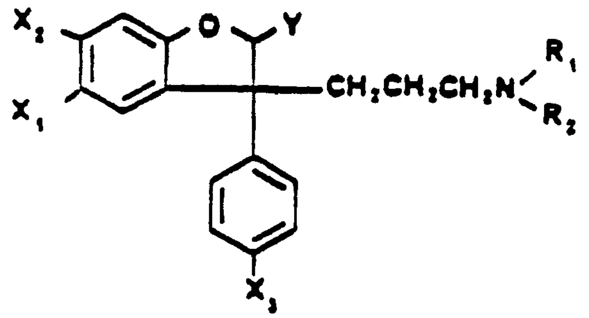

(I)

dans laquelle:

- $X_1$ et $X_2$, identiques ou différents, représentent chacun un atome d'hydrogène ou d'halogène, un radical alcoxy renfermant de 1 à 4 atomes de carbone, ou forment ensemble un groupe méthylènedioxy,
- $X_3$ représente un atome d'hydrogène ou un radical alkyle de 1 à 4 atomes de carbone,
- Y représente un atome d'hydrogène ou un radical hydroxy,
- $R_1$ et $R_2$ identiques ou différents représentent chacun un radical alkyle de 1 à 4 atomes de carbone, en chaîne droite ou ramifiée, à condition toutefois que quand Y représente un atome d'hydrogène $X_1$ ou $X_2$ ou $X_1$ et $X_2$ simultanément, représentent un radical alcoxy renfermant de 1 à 4 atomes de carbone,
  - ou bien
    $R_1$ est un méthyle et,
    $R_2$ représente un groupement phénylalkyle renfermant de 7 à 9 atomes de carbone ou un groupe indanyle-2, éventuellement substitués sur le cycle aromatique par un ou deux radicaux alcoxy renfermant de 1 à 4 atomes de carbone, à condition toutefois que $R_2$ ne représente jamais un phénéthyle quand $X_1$ et $X_2$ représentent chacun un atome d'hydrogène ou d'halogène et Y représente un atome d'hydrogène,
  - ou bien
    $R_1$ et $R_2$ forment ensemble, avec l'atome d'azote auxquels ils sont attachés, un radical morpholinyle-4, un radical tétrahydro-1,2,6,7 thiéno (2,3-c) pyridinyle-1, un radical tétrahydroisoquinolyle-2 éventuellement substitué par un ou deux radicaux alcoxy de 1 à 4 atomes de carbone, un groupe alkyl-4 (de 1 à 4 atomes de carbone) pipérazinyle-1, ou un groupe phényl-4 piperazinyle-1 éventuellement substitué au niveau du cycle aromatique par 1 ou 2 radicaux alcoxy renfermant de 1 à 4 atomes de carbone,

sous forme racémique ou d'isoméres optiques
et leurs sels d'addition à un acide minéral ou organique pharmaceutiquement acceptable.

La présente invention a également pour objet le procédé de préparation des composés de formule générale I, caractérisé en ce que l'on condense une aryl-3 (chloro-3 propyl)-3 benzofurannone-2 de formule générale II:

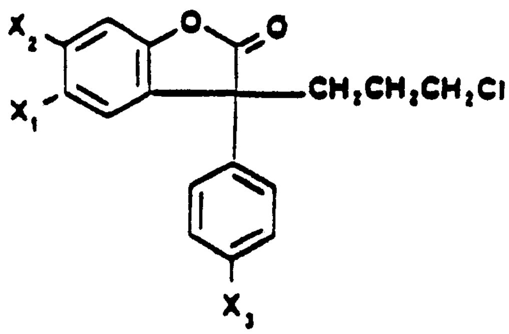

(II)

dans laquelle la définition des substituants $X_1$, $X_2$, $X_3$ demeure celle mentionnée précédemment pour la formule générale I,

avec une amine secondaire de formule générale III:

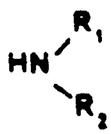

(III)

dans laquelle la définition de $R_1$ et $R_2$ demeure celle indiquée précédemment pour la formule générale I, en un dérivé de formule générale IV:

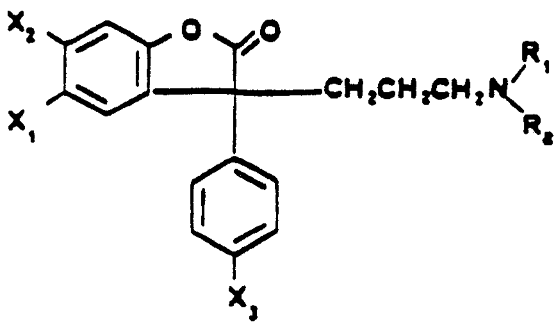

(IV)

dans laquelle $X_1$, $X_2$, $X_3$, $R_1$, $R_2$ ont les significations précédemment définies pour la formule générale I, qui est ensuite,

- soit réduit partiellement en un dérivé de formule générale I':

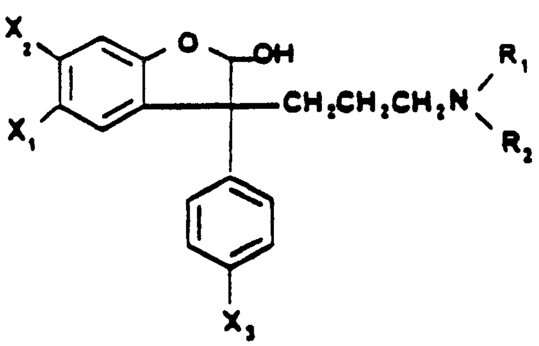

(I')

dans laquelle $X_1$, $X_2$, $X_3$, $R_1$ et $R_2$ sont tels que précédemment définis pour la formule générale I,

- soit ouvert par réduction en un diol de formule générale V:

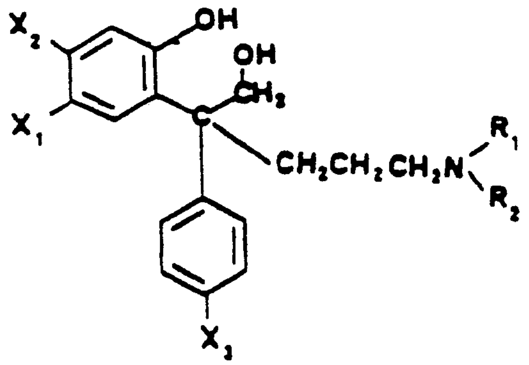

(V)

dans laquelle $X_1$, $X_2$, $X_3$, $R_1$, $R_2$ ont les significations précédemment définies pour la formule générale I, qui est finalement cyclisé en un dérivé de formule générale I'':

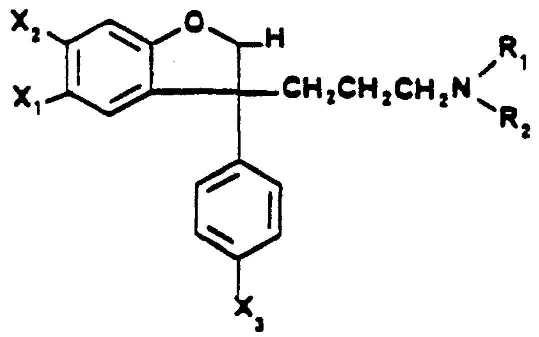

(I")

dans laquelle la signification de $X_1$, $X_2$, $X_3$, $R_1$ et $R_2$ est identique à celle de la formule générale I.

Une variante de ce procédé consiste à soumettre l'aryl-3, (chloro-3 propyl)-3 benzofurannone-2 de formule générale II à l'action d'un réducteur pour obtenir un diol de formule générale VI:

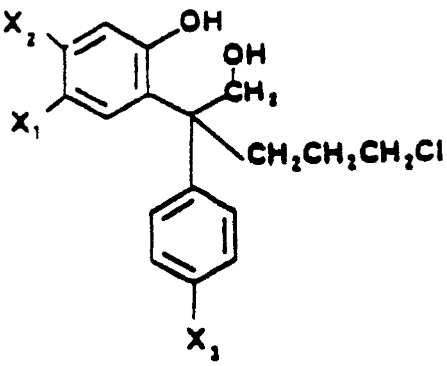

(VI)

dans laquelle la définition de $X_1$, $X_2$, $X_3$ demeure celle indiquée précédemment dans la formule générale I, et qui est ensuite cyclisé en un dérivé de formule générale VII:

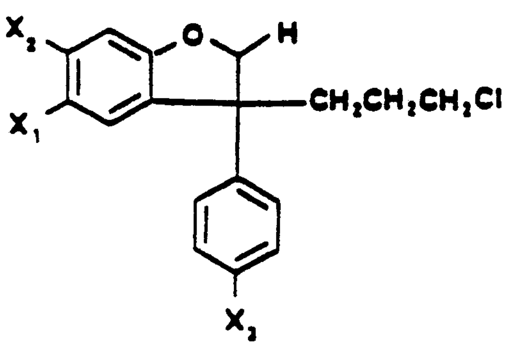

(VII)

dans laquelle la définition de $X_1$, $X_2$, $X_3$ a les significations précédemment définies pour la formule générale I

et que l'on condense avec une amine de formule générale III pour obtenir un composé de la formule

générale I''.

L'ensemble des composés de formule I' et I'' forme l'ensemble des composés de formule I, qui peuvent être, si l'on désire

- soit transformés en leurs sels d'addition à un acide minéral ou organique pharmaceutiquement acceptable.
- soit séparés en leurs isomères optiques, puis éventuellement salifiés par des acides.

Les matières premières de formule générale II peuvent être préparées selon la méthode décrite par Zaugg H et al. (J. Org. Chem. (1961) 26, 4821-4828).

La condensation des amines secondaires de formule générale III avec les composés de formule générale II ou VII s'effectue de préférence dans un solvant organique polaire, tel que l'éthanol ou la méthyléthylcétone à une température comprise entre 50° et 90°C, en présence de sels minéraux tels que le carbonate de sodium ou l'iodure de sodium.

Les diols V et VI sont obtenus respectivement par réduction et hydrolyse des lactones IV et II. La réduction des lactones est de préférence réalisée à l'aide d'hydrures métalliques dans un solvant organique inerte à température ambiante, ou légèrement supérieure. Plus précisemment, on peut utiliser comme réducteur un hydrure métallique tel que l'hydrure double de lithium et d'aluminium, dans le tétrahydrofuranne.

La réduction partielle des lactones de formule générale IV, s'effectue dans un solvant organique aprotique, par exemple le toluène, à une température comprise entre -50°C et -80°C en présence d'hydrure de diisobutylaluminium.

La cyclisation des alcool-phénols V et VI est effectuée en traitant ces composés par le butyllithium dans un solvant inerte anhydre comme le tétrahydrofuranne, puis par un excès de chlorure de paratoluènesulfonyle, ou par d'autres méthodes déjà connues dans la littérature, décrites par Padwa A., An A., Owens W., dans J. Org. Chem. (1978), 43, N° 2,303-309 et par Gervais C., Anker D., Carret G. et Pacheco H., dans Tetrahedron (1979), 33, 745-752.

Tous les dérivés nouveaux, faisant partie de cette invention, peuvent être purifiés par des méthodes physiques telles que la cristallisation ou la chromatographie.

La présente invention a également pour objet les isomères optiques des dérivés répondant à la formule générale I. Ces isomères peuvent être préparés par dédoublement des composés racémiques. Comme agent de dédoublement, on peut citer par exemple, les acides (+) et (-) dibenzoyltartriques.

Parmi les acides pharmaceutiquement acceptables pour la préparation des sels d'addition aux composés de formule générale I, on peut citer les acides phosphorique, chlorhydrique, citrique, oxalique, sulfurique, tartrique, maléïque, etc...;

Les composés selon l'invention, ainsi que leurs sels sont doués de propriétés pharmacologiques fort intéressantes et se distinguent des autres dérivés du dihydro-2,3 benzofuranne déjà connus.

En effet, les essais pharmacologiques in vitro ont montré que ces composés sont de puissants modulateurs des mouvements intracellulaires et transmembranaires du calcium. Certaines activités cellulaires des muscles lisses ou striés, notamment leur contractilité, sont liées à la concentration intracytoplasmique du calcium, et la perturbation de cette concentration a pu être mise en évidence dans certaines affections faisant intervenir la contractilité musculaire (angor, hypertension artérielle, asthme, migraine, spasmes oesophagiens).

Le calcium a également un rôle dans la régulation du métabolisme cellulaire, en particulier mitochondrial, et ce métabolisme est perturbé dans des maladies comme l'ischémie cardiaque ou cérébrale.

La propriété des composés de l'invention de moduler les mouvements du calcium permet donc son application dans le traitement de l'hypertension, de l'angor, de l'asthme, des spasmes oesophagiens, de la migraine ou de l'ischémie myocardique et cérébrale. (Burger's Medicinal Chemistry 4ème-Ed. Part. III, p. 54-56, John Wiley and Sons Inc., USA, 1981).

Les essais pharmacologiques chez le chien, ont prouvé in vivo, que l'activité des composés de l'invention est au moins 20 fois supérieure par rapport à celle des autres dérivés du dihydro-2,3 benzofuranne déjà connus (Brevet US N° 3 156 688) et ont ainsi confirmé le grand intérêt de leur emploi en thérapeutique.

L'invention s'étend aussi aux compositions pharmaceutiques renfermant comme principe actif au moins un composé de formule générale I l'un de ses isomères ou l'un de ses sels d'addition avec un acide minéral ou organique pharmaceutiquement compatible en association avec un ou plusieurs excipients, inertes, non toxiques et appropriés.

Les compositions pharmaceutiques ainsi obtenues sont présentées avantageusement sous des formes diverses telles que par exemple, comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie d'administration. La voie d'administration préférée est la voie buccale ou parentérale. D'une manière générale, la posologie unitaire s'échelonnera entre 1 et 300 mg et la posologie journalière, utilisable en thérapeutique humaine ou animale entre 1 et 900 mg.

Les exemples suivants, donnés à titre non limitatif, illustrent l'invention.

Les points de fusion indiqués sont mesurés selon la technique micro-Kofler. Les spectres de résonance magnétique nucléaire du proton (RMN) ont généralement été enregistrés en utilisant le $CDCl_3$ comme solvant, et le TMS comme référence interne. Les spectres infra-rouge sont obtenus avec des suspensions de produits dans le Nujol.

**Exemple 1:**

Phosphate du méthoxy-5 phényl-3 [(N-(diméthoxy-3,4 phénéthyl) N-méthylamino)-3 propyl] -3 dihydro-2,3 benzofuranne

a) Méthoxy-5 phényl-3 [(N-(diméthoxy-3,4 phénéthyl) N-méthyl-amino)-3 propyl]-3 benzofurannone-2.

Un mélange contenant 16,5 g de méthoxy-5 (chloro-3 propyl)-3 benzofurannone-2, préparée selon la méthode décrite par Zaugg H et al. (J. Org. Chem. (1961), 26, 4821-4828), 12,2 g de N-méthylhomovératrylamine, 20 g de carbonate de sodium et 1,6 g d'iodure de sodium dans 500 ml d'éthylméthylcetone est chauffé à reflux, sous agitation pendant 24 heures. Après refroidissement, les sels minéraux sont éliminés par filtration et le filtrat huileux, après condensation est repris dans 450 ml d'un mélange d'eau et de dichlorométhane (1 : 3). Après décantation, la phase organique est récupérée et la phase aqueuse est réextraite avec 300 ml de dichlorométhane. Les phases organiques sont rassemblées, évaporées sous pression réduite. On obtient, 25 g d'une huile qui est ensuite chromatrographiée sur colonne de silice, en utilisant un gradient de solvant (dichlorométhane pur, puis de mélanges de dichlorométhane avec 5 %, 10 % et 25 % d'acétone) comme éluant.

Après évaporation du solvant, l'huile pure obtenue (20 g) est utilisée telle quelle dans l'étape suivante. Ses constantes physiques sont mentionnées dans le tableau 1.

Rendement 75 %.

b) Phényl-2 [(hydroxy-2 méthoxy-5) phényl]-2 [N-(diméthoxy-3,4 phénéthyl) N-méthylamino]-5, pentanol-1.

A une suspension d'hydrure double de lithium et d'aluminium dans 200 ml de tétrahydrofuranne, sont ajoutés à une température comprise entre 25°C et 35°C, par petites quantités, 20 g de la lactone obtenue précédemment en solution dans 300 ml de tétrahydrofuranne. Après 30 minutes d'agitation, le mélange réactionnel est hydrolysé à la température de 0°C, successivement par 40 ml d'éthanol, 20 ml d'eau, 40 ml d'une solution d'hydroxyde de sodium à 50 % et 60 ml d'eau. Le précipité formé est éliminé par filtration et le filtrat obtenu est concentré.

On obtient 17,6 g d'une huile pure, dont les constantes physiques sont décrites dans le tableau 2.

Rendement 87 %.

c) Méthoxy-5 phényl-3 [(N-(diméthoxy-3,4 phénéthyl) N-méthylamino)-3 propyl] -3 dihydro-2,3 benzofuranne.

Le diol obtenu précédemment (15,3 g) est dissous dans 200 ml de tétrahydrofuranne anhydre. A cette solution sont ajoutés lentement, à la température de 0°C, 42 ml d'une solution 0,067 M de butyllithium dans l'hexane. La température de la réaction est maintenue pendant 30 minutes à 10° - 15°C, et ensuite, sont introduits en refroidissant et sous agitation 12,2 g de chlorure de paratoluènesulfonyle en solution dans 50 ml de tétrahydrofuranne. L'agitation est poursuivie pendant deux heures à la température ambiante.

Le milieu réactionnel est ensuite hydrolysé par 100 ml d'eau et concentré sous pression réduite. Le résidu obtenu est repris par 300 ml de dichlorométhane, lavé soigneusement avec une solution aqueuse de carbonate de sodium à 10 %, puis avec de l'eau. La phase organique est séchée sur sulfate de sodium anhydre, concentrée et chromatographiée sur colonne de silice. L'éluant utilisé est un mélange d'éther éthylique, d'acétone, d'hexane et de méthanol (30/20/40/10).

4,8 g d'huile pure sont ainsi obtenus.

Rendement 30 %.

Le phosphate de méthoxy-5 phényl-3 [(N-(diméthoxy-3,4 phénéthyl) N-méthylamino)-3 propyl]-3 dihydro-2,3 benzofuranne est obtenu après addition de 0,01 mole d'acide phosphorique à l'huile obtenue précédemment et recristallisation dans un mélange d'acétone et d'éther éthylique (20/80).

P.F. = 70°C.

Les constantes physiques spectrales de ce composé sont indiquées dans le tableau 3.

**Exemple 2a - 10a:**

Les dérivés suivants ont été préparés selon le procédé décrit dans l'exemple 1a. Leurs constantes physiques sont indiquées dans le tableau 1.

2a Chloro-5 phényl-3 [((méthoxy-3 phényl)-4 pipérazinyl-1)-3 propyl]-3 benzofurannone-2.
Rendement 90 %.

3a Diméthoxy-5,6 phényl-3 [(N-(diméthoxy-3,4 phénéthyl) N-méthylamino)-3 propyl]-3 benzofurannone-2.
Rendement 60 %.

4a Chloro-5 phényl-3 [(N-(diméthoxy-3,4 phénéthyl) N-méthylamino)-3 propyl]-3 benzofurannone-2
Rendement 53 %.

5a Méthoxy-5 (méthyl-4 phényl)-3 [(N-(diméthoxy-3,4 phénéthyl) N-méthylamino)-3 propyl]-3 benzofurannone-2
Rendement 50 %.

6a Méthoxy-5 phényl-3 [(N-(diméthoxy-5,6 indanyl-2) N-méthylamino)-3 propyl]-3 benzofurannone-2.
Rendement 70 %.

7a Méthoxy-5 phényl-3 [(diméthoxy-6,7 tétrahydro-1,2,3,4 isoquinolyl-2)-3 propyl]-3 benzofurannone-2.
Rendement 65 %.

8a Méthoxy-5 phényl-3 [(N-phénéthyl N-méthylamino)-3 propyl]-3 benzofurannone-2.
Rendement 70 %.

9a Méthoxy-5 phényl-3 [(méthyl-4 pipérazinyl-1)-3 propyl]-3 benzofurannone-2
Rendement 75 %.

10a Méthoxy-5 phényl-3 [(N-diéthylamino)-3 propyl]-3 benzofurannone-2
Rendement 80 %.

**Exemples 2b - 10b**

Les composés suivants ont été préparés à partir des benzofurannones-2 correspondantes décrites ci-dessus, selon le procédé décrit dans l'exemple 1b. Les constantes physiques spectrales de ces alcools sont indiquées dans le tableau 2.

2b Phényl-2 [(hydroxy-2 chloro-5) phényl]-2 [(méthoxy-3 phényl)-4 piperazinyl-1]-5 pentanol-1.
F = 120° C
Rendement 98 %.

3b Phényl-2 [(hydroxy-2 diméthoxy-4,5) phényl]-2 [N-(diméthoxy-3,4 phénéthyl) N-méthylamino]-5 pentanol-1
Rendement 40 %.

4b Phényl-2 [(hydroxy-2 chloro-5) phényl]-2 [N-(diméthoxy-3,4 phénéthyl) N-méthylamino]-5 pentanol-1
Rendement 70 %.

5b (méthyl-4 phényl)-2 [(hydroxy-2 méthoxy-5) phényl]-2 [N-(diméthoxy-3,4 phénéthyl) N-méthylamino]-5 pentanol-1.
Rendement 95 %.

6b Phényl-2 [(hydroxy-2 méthoxy-5) phényl]-2 [N-(diméthoxy-5,6 indanyl-2) N-méthylamino]-5 pentanol-1
Rendement 90 %.

7b Phényl-2 [(hydroxy-2 méthoxy-5) phényl]-2 (diméthoxy-6,7 tétrahydro-1,2,3,4 isoquinolyl-2)-5 pentanol-1
Rendement 60 %.

8b Phényl-2 [(hydroxy-2 methoxy-5) phényl]-2 (N-phénéthyl N-méthylamino)-5 pentanol-1
Rendement 70 %.

9b Phényl-2 [(hydroxy-2 méthoxy-5) phényl]-2 (méthyl-4 pipérazinyl-1)-5 pentanol-1
Rendement 65 %.

10b Phényl-2 [(hydroxy-2 méthoxy-5) phényl]-2 (N-diéthylamino)-5 pentanol-1.
Rendement 90 %.

**Exemples 2 - 10**

Les dihydro-2,3 benzofurannes suivantes (exemples 2 - 10) ont été préparées par cyclisation des composés 2b - 10b selon le procédé décrit dans l'exemple 1c. Leurs constantes physiques spectrales sont indiquées dans le tableau 3.

2 Chloro-5 phényl-3 [((méthoxy-3 phényl)-4 pipérazinyl-1)-3 propyl]-3 dihydro-2,3 benzofuranne.
Rendement 30 %.
Point de fusion de l'oxalate: 140° C

3 Diméthoxy-5,6 phényl-3 [(N-(diméthoxy-3,4 phénéthyl) N-méthylamino)-3 propyl]-3 dihydro-2,3 benzofuranne.
Rendement 50 %.
Point de fusion du phosphate: 94° C

4 Chloro-5 phényl-3 [(N-(diméthoxy-3,4 phénéthyl) N-méthylamino)-3 propyl]-3 dihydro-2,3 benzofuranne.
Rendement 52 %.
Point de fusion du chlorhydrate: 95° C

5 Méthoxy-5 (méthyl-4 phényl)-3 [(N-(diméthoxy-3,4 phénéthyl) N-méthylamino)-3 propyl]-3 dihydro-2,3 benzofuranne.
Rendement 50 %.

Point de fusion du phosphate: 84° C

6 Méthoxy-5 phényl-3 [(N-(diméthoxy-5,6 indanyl-2) N-méthylamino)-3 propyl]-3 dihydro-2,3 benzofuranne.
Rendement 40 %.
Point de fusion du phosphate: 110° C

7 Méthoxy-5 phényl-3 [(diméthoxy-6,7 tetrahydro-1,2,3,4 isoquinolyl-2)-3 propyl]-3 dihydro-2,3 benzofuranne.
Rendement 60 %.
Point de fusion du phosphate: 106° C

8 Méthoxy-5 phényl-3 [(N-phénéthyl N-méthylamino)-3 propyl]-3 dihydro-2,3 benzofuranne.
Rendement 55 %.
Point de fusion du phosphate: 75° C

9 Méthoxy-5, phényl-3, [(méthyl-4, piperazinyl-1)-3 propyl] -3 dihydro-2,3 benzofuranne.
Rendement 55 %.
Point de fusion du chlorhydrate: 175° C

10 Méthoxy-5 phényl-3 [(N-diéthylamino)-3 propyl]-3 dihydro-2,3 benzofuranne.
Rendement 30 %.
Point de fusion du chlorhydrate: 146° C

**Exemple 11**

Phosphate d'hydroxy-2 méthoxy-5 phényl-3 [(N-(diméthoxy-3,4 phénéthyl) N-méthylamino) -3 propyl]-3 dihydro-2,3 benzofuranne.

11a <u>Hydroxy-2 méthoxy-5 phényl-3 [(N-(diméthoxy-3,4 phénéthyl) N-méthylamino)-3 propyl]-3 dihydro-2,3 benzofuranne</u>

A une solution de 200 ml de toluène, refroidie à -80° C et contenant 4,6 g de la lactone obtenue précédemment dans l'exemple 1a, sont ajoutés lentement sous agitation, 41 ml d'une solution molaire d'hydrure de diisobutyaluminium dans le toluène. Le mélange réactionnel est laissé sous agitation à -80° C pendant 3 heures, puis sont ajoutés 80 ml d'une solution 2M d'isopropanol dans le toluène en laissant la température augmenter et arriver à 0° C. Ensuite, 8 ml d'eau et 20 g de silice fine sont introduits dans le milieu réactionnel.

Après filtration sur silice et concentration, le résidu est repris par 300 ml de dichlorométhane lavé à l'eau, séché sur sulfate de sodium anhydre et reconcentré. 3,3 g d'huile pure sont ainsi obtenus.

Rendement 70 %.

Le phosphate d'hydroxy-2 méthoxy-5 phényl-3 [(N-(diméthoxy-3,4 phénéthyl) N-méthylamino) -3 propyl] -3 dihydro-2,3 benzofuranne est préparé par addition d'une quantité équimolaire d'acide phosphorique et recristallisation dans un mélange d'éther éthylique et d'acétone (95/5). Les constantes physiques spectrales du composé sont indiquées dans le tableau 3.

F = 108° C.

**Exemple 12**

Phosphate d'hydroxy-2 diméthoxy-5,6 phényl-3 [(N-(diméthoxy-3,4 phénéthyl) N-méthylamino)-3 propyl]-3 dihydro-2,3 benzofuranne.

Ce composé est préparé à partir de la lactone de l'exemple 3a et selon le procédé décrit dans l'exemple 11. Rendement 40 %. Ses constantes physiques spectrales sont indiquées dans le tableau 3.

F = 100° C.

**Exemple 13**

Phosphate de phényl-3 [N-(diméthoxy-3,4 phénéthyl) N-méthylamino)-3 propyl]-3 dihydro-2,3 benzofuranne.

13a Phényl-2 (hydroxy-2 phényl)-2 chloro-5 pentanol-1.

A une suspension d'hydrure double de lithium et d'aluminium dans 250 ml d'éther sont ajoutés à la température ambiante, très lentement et successivement, 250 ml d'une solution d'éther éthylique contenant 40 g de chlorure d'aluminium, ensuite un mélange contenant 40 g de (chloro-3 propyl)-3

benzofurannone-2 et 18,6 g de chlorure d'aluminium dans 1 l d'éther éthylique.

Le milieu réactionnel est laissé sous agitation pendant 1 heure et ensuite est versé sur 1,5 l d'acide chlorhydrique 1N glacé. Après extraction par 500 ml d'éther, la phase organique est récupérée, lavée avec 500 ml d'une solution aqueuse saturée de chlorure de sodium, séchée sur sulfate de sodium anhydre et évaporée. Le résidu est recristallisé dans un mélange d'éther de pétrole et d'éther éthylique (70/30).

On obtient 28,5 g d'alcool.
Rendement 70 %
F = 119°C
Spectre RMN du proton: 1,2 à 1,9 ppm m 2H;
2,1 à 2,6 ppm m 3H et 1H échangeable;
3,4 ppm t 2H;
4,1 ppm q 2H;
6,45 ppm 1H échangeable;
6,6 à 7,6 ppm m 9H.

13b Phényl-3 (chloro-3 propyl)-3 dihydro-2,3 benzofuranne.

A 7 g du diol obtenu précédemment refroidi à la température de -80°C, sont ajoutés très lentement 0,051 moles de butyllithium en solution dans 31,9 ml d'hexane. La réaction se poursuit pendant 30 minutes à la température de -80°C et ensuite pendant 15 minutes à la température de -65°C. La solution réactionnelle est ensuite refroidie à -80°C et sont ajoutés 6,95 g de chlorure de paratoluènesulfonyle en solution dans 80 ml de tétrahydrofuranne. La température est portée lentement à 25°C. Le milieu réactionnel est laissé pendant 1 heure sous agitation et ensuite hydrolysé par 110 ml d'une solution d'hydroxyde de sodium 4N.

Après deux heures de contact, la solution est concentrée et le résidu est récupéré dans l'éther éthylique. La phase organique est lavée à l'eau, séchée sur sulfate de sodium anhydre et concentrée. L'huile obtenue est purifiée sur colonne de silice par élution avec 1,5 l d'un mélange d'éther éthylique et d'hexane (10/90). 5,3 g de phényl-3 (chloro-3 propyl)-3 dihydro-2,3 benzofuranne sont ainsi obtenus.

Rendement 80 %.
Spectre RMN du proton:
1,5 à 1,9 ppm m 2H;
2,2 à 2,3 ppm m 2H;
3,48 ppm t 2H;
4,53 ppm s 2H;
6,8 à 7,4 ppm m 9H.

13c Phényl-3 [(N-(diméthoxy-3,4 phénéthyl) N-méthyl-amino)-3 propyl-] 3 dihydro-2,3 benzofuranne.

5,2 g du composé obtenu précédemment dans l'exemple 15b sont portés à reflux pendant 4 heures dans 250 ml d'éthylméthylcétone en présence de 4,5 g de N-méthyl homovératrylamine, 8 g de carbonate de sodium et 0,5 g d'iodure de sodium.

Après élimination des sels minéraux par filtration, le milieu réactionnel est concentré, repris dans 150 ml d'une solution aqueuse de carbonate de sodium à 10 %, et ensuite extrait au benzène.

La phase organique est séchée sur sulfate de sodium anhydre, concentrée et le résidu obtenu purifié sur une colonne de silice sous pression. Après élution avec un mélange de dichlorométhane, acétone et méthanol (67/30/3). On obtient 5,4 g de produit pur.

Rendement 50 %.

Le phosphate de phényl-3 [(N-(diméthoxy-3,4 phénéthyl) N-méthylamino)-3 propyl ] -3 dihydro-2,3 benzofuranne est obtenu après addition d'une quantité appropriée d'acide phosphorique à la base obtenue dans l'exemple 13c. Le sel a été purifié par cristallisation dans un mélange d'éther éthylique et d'éthanol (95/5). Les constantes physiques spectrales du composé sont décrites dans le tableau 3.

F = 70°C.

**Exemples 14 - 16**

Les composés suivants (exemples 14 - 16) sont obtenues par condensation du phényl-3 (chloro-3 propyl)-3 dihydro-2,3 benzofuranne (exemple 13b) avec des amines secondaires adéquates selon le procédé décrit dans l'exemple 13c. Les constantes physiques spectrales des composés 14-16 sont indiquées dans le tableau 3.

14 Phényl-3 [(tétrahydro-1,2,6,7 thieno-(2,3-c) pyridinyl-1)-3 propyl]-3 dihydro-2,3 benzofuranne.
Rendement 70 %.
Point de fusion du citrate: 90°C

15 Phényl-3 [(morpholinyl-4)-3 propyl]-3 dihydro-2,3 benzofuranne.
Rendement 40 %.

Point de fusion du chlorhydrate: 200° C

16 phényl-3 [((méthoxy-3 phényl)-4 pipérazinyl-1)-3 propyl]-3 dihydro-2,3 benzofuranne.
Rendement 40 %.
Point de fusion du chlorhydrate: 171° C

Les constantes physiques spectrales de ces composés sont décrites dans le tableau 3.

**Etude pharmacologique des composés de l'invention**

**Exemple 17**

Evaluation de l'activité inhibitrice de la contraction sur l'artère coronaire de chien

L'activité inhibitrice de la contraction induite par les ions calciques a été évaluée in vitro sur l'artère coronaire du chien selon le procédé décrit par Godfraind et Miller dans Circ. Res. (1983) 52, N° 2, p. 81-91. La contraction a été induite par une solution contenant 100 mM d'ion potassium. Les composés à examiner ont été ensuite introduits à doses cumulées, et après un temps d'action de 15 minutes, la préparation a été réactivée par des solutions d'une concentration de 10 et 20 mM en ions calciques. La dose capable d'inhiber 50 % ($ED_{50}$), de la contraction maximale a été évaluée. Les $ED_{50}$ mesurées des différents composés sont indiquées dans le tableau 4 (Voir tableau ci-après).

**Tableau 4**

| Composés de l'exemple | $ED_{50}$ (M) |
|---|---|
| 1 | $1,7.10^{-6}$ |
| 2 | $1,0.10^{-5}$ |
| 3 | $9,0.10^{-5}$ |
| 4 | $3,0.10^{-5}$ |
| 5 | $2,6.10^{-5}$ |
| 6 | $1,0.10^{-5}$ |
| 7 | $6,3.10^{-6}$ |
| 8 | $8,4.10^{-6}$ |
| 9 | $3,5.10^{-5}$ |
| 10 | $6,1.10^{-6}$ |
| 11 | $6,2.10^{-6}$ |
| 12 | $8,0.10^{-6}$ |
| 13 | $4,0.10^{-6}$ |
| 14 | $9,0.10^{-6}$ |
| 15 | $5,0.10^{-5}$ |
| 16 | $9,0.10^{-6}$ |

**Exemple 18**

Evaluation de l'activité inhibitrice de la contraction sur l'artère caudale de rat

L'activité inhibitrice de la contraction sur les vaisseaux a été mesurée in vitro sur l'artère caudale du rat Wistar mâle, perfusée à débit constant et préalablement dénervée par une solution de 6-hydroxydopamine. Après vasoconstrictions induites par une solution contenant 90 mM d'ions potassium, la pression du liquide de la perfusion au niveau de l'entrée dans l'artère a été mesurée avant et après l'addition des composés à examiner à des doses cumulées selon la méthode décrite par Worcel M. dans J. Pharmacol. Exp. Ther., (1978), 207, 320-330. La dose capable d'inhiber 50 % ($ED_{50}$) de la contraction maximale a été évaluée. Les $ED_{50}$ des composés de l'invention sont comprises entre $1.10^{-5}$ et $1.10^{-6}$M.

**Exemple 19**

Evaluation de l'activité inhibitrice de la contraction et du rythme sur l'oreillette de rat.

L'activité inhibitrice de la concentration et du rythme a été évaluée in vitro sur l'oreillette gauche isolée du rat Wistar albinos mâle, selon la méthode décrite par Refsum H. et Landmark K. dans Acta Pharmacol. (1975), 37, 369-379. L'organe a été soumis à une stimulation électrique par électrodes bipolaires de platine, avec des pulsations d'une durée de 0,5 msec, et une fréquence de 3 Hz. La tension initiale imposée (500 mg)

correspondait au maximum de la contraction. La contraction isométrique et le rythme ont été mesurés avant et après l'addition à des doses cumulées des composés à examiner. La dose capable d'inhiber 50 % ($ED_{50}$) de la contraction maximale a été évaluée. Les $ED_{50}$ des composés de l'invention sont comprises entre $1.10^{-5}$ et $1.10^{-4}$M.

**Exemple 20**

Effets hémodynamiques in vivo:

Les effets hémodynamiques ont été évalués chez le chien batard de 25 à 30 kg anesthésié au phenobarbital sodique, thoracotomie au 5ème espace intercostal gauche, portant des bagues électromagnétiques au niveau de la branche circonflexe de l'artère coronaire gauche et au niveau de l'aorte ascendante. L'effet bradycardisant maximum, (E.B. max.) les résistances coronaires moyennes (C.V.R.M.), le débit coronaire (C.B.F.M.) et la pression artérielle (P.A.) ont été mesurés après une injection d'une dose de 300 µg/kg et 1000 µg/kg par voie intraveineuse des composés des exemples N° 1 et 13.

Le Tableau 5 résume les différents résultats exprimés en pourcentage d'augmentation (+ %) ou de diminution (- %) des valeurs par rapport à celles observées avant traitement.

**Tableau 5**

| Composés de l'exemple | Dose | EB max | CRVM | CBFM | PA |
|---|---|---|---|---|---|
| Exemple 1 | 300 µg/kg | - 7 % | - 39 % | + 51 % | - 9 % |
| (phosphate) | 1000 µg/kg | - 13 % | - 57 % | + 85 % | - 21 % |
| Exemple 13 | 300 µg/kg | - 8 % | - 36 % | + 31 % | - 16 % |
| (phosphate) | 1000 µg/kg | - 10 % | - 60 % | + 59 % | - 31 % |

**Préparation pharmaceutique**

La préparation pharmaceutique suivante est donnée à titre d'exemple non limitatif.

<u>Phosphate du méthoxy-5 phényl-3 [(N-(diméthoxy-3,4 phénéthyl) N-méthylamino)-3 propyl]-3 dihydro-2,3 benzofuranne</u> exprimé en:

| | |
|---|---|
| base | 25,00 g |
| amidon de blé | 100,00 g |
| amidon de maïs | 80,00 g |
| stéréate de magnésium | 15,00 g |
| talc | 20,00 g |

pour 1000 comprimés à 25 mg de principe actif.

TABLEAU 1 : COMPOSES DE FORMULE GENERALE IV

| EX | $X_1$ | $X_2$ | $X_3$ | $R_1$ | $R_2$ | IR ν(CO) lactone | RMN |
|---|---|---|---|---|---|---|---|
| 1a | $-OCH_3$ | -H | -H | $-CH_3$ | 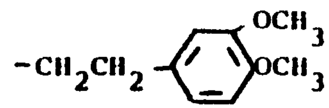 | 1800 $cm^{-1}$ | 0,8 à 2 ppm m 2H ; 2 à 3 ppm m 8H ; 2,2 ppm s 3H ; 3,9 ppm s 9H ; 6,5 à 7,5 ppm m 11H. |
| 2a | -Cl | -H | -H | | 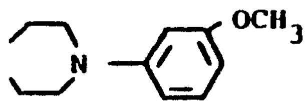 | 1810 $cm^{-1}$ | 1,4 ppm m 2H ; 2 à 2,8 ppm m 6H ; 2,8 à 3,4 ppm m 4H ; 3,7 ppm s 3H ; 6,2 à 7,6 ppm m 12H. |
| 3a | $-OCH_3$ | $-OCH_3$ | -H | $-CH_3$ | 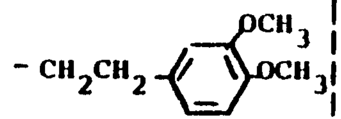 | 1790 $cm^{-1}$ | 1,0 à 1,8 ppm m 2H ; 2 à 3 ppm m 11H ; 3,9 à 3,95 ppm s 12H ; 6,8 ppm m 5H ; 7,4 ppm m 5H. |
| 4a | -Cl | -H | -H | $-CH_3$ | 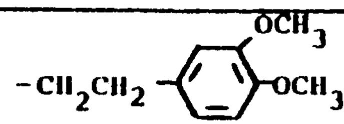 | 1805 $cm^{-1}$ | 1 à 2 ppm m 2H ; 2 à 3 ppm m 11H ; 3,9 ppm s 6H ; 6,5 à 7,6 ppm m 11H. |
| 5a | $-OCH_3$ | -H | $-CH_3$ | $-CH_3$ | 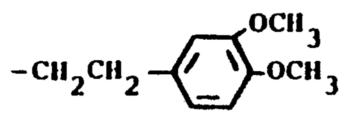 | 1800 $cm^{-1}$ | 1,1 à 1,5 ppm m 2H ; 2,1 à 2,7 ppm m 8H ; 2,1 ppm s 3H ; 2,3 ppm s 3H ; 3,75 ppm s 9H ; 6,7 à 7,4 ppm m 10H. |
| 6a | $-OCH_3$ | -H | -H | $-CH_3$ | 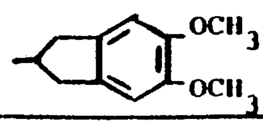 | 1800 $cm^{-1}$ | 1 à 4 ppm m 14H ; 3,9 s 9H ; 6,5 à 7,8 m 10H. |

**TABLEAU 1 (suite N° 1) : COMPOSES DE FORMULE GENERALE IV**

| EX | $X_1$ | $X_2$ | $X_3$ | $R_1$ | $R_2$ | IR ν (CO) lactone | RMN |
|---|---|---|---|---|---|---|---|
| 7a | $-OCH_3$ | -H | -H | | 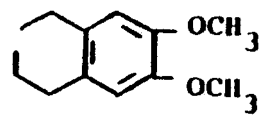 | 1790 $cm^{-1}$ | 1 à 2 ppm m 2H ; 2 à 3 ppm m 8H ; 3,4 ppm s 2H ; 3,8 ppm s 9H ; 6,4 à 7,6 ppm m 10H. |
| 8a | $-OCH_3$ | -H | -H | $-CH_3$ | $-CH_2CH_2-$ 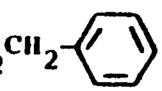 | 1790 $cm^{-1}$ | 1 à 1,5 ppm m 2H ; 2 à 2,8 ppm m 11H ; 3,8 ppm s 3H ; 6,5 à 7,5 ppm m 13 H. |
| 9a | $-OCH_3$ | -H | -H | | 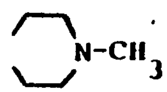 | 1800 $cm^{-1}$ | (chlorhydrate) 1 à 3 ppm m 4H ; 2,8 ppm s 11H ; 3,8 ppm s 3H ; 3,8 ppm s 3H ; 6,7 à 7,8 ppm m 8H ; 11 à 13 ppm 2H échangeables. |
| 10a | $-OCH_3$ | -H | -H | $-C_2H_5$ | $-C_2H_5$ | 1800 $cm^{-1}$ | 0,8 à 2 ppm m 8H ; 2 à 2,6 ppm m 8H ; 3,8 ppm s 3H ; 6,7 à 7,5 ppm m 8H. |

**TABLEAU 2 : COMPOSES DE FORMULE GENERALE V**

| EX | $X_1$ | $X_2$ | $X_3$ | $R_1$ | $R_2$ | IR ν(OH) | RMN |
|---|---|---|---|---|---|---|---|
| 1b | $-OCH_3$ | -H | -H | $-CH_3$ | 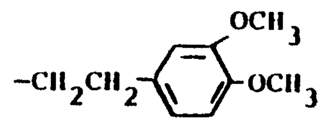 | 3600-2300 $cm^{-1}$ | 1 à 3 ppm m 4H ; 2,4 ppm s 3H ; 2,8 ppm m 6H ; 3,8 à 3,9 ppm m 9H ; 3,9 ppm q 2H ; 6,2 ppm 2H échangeables ; 6,8 à 7,35 ppm m 11H. |
| 2b | -Cl | -H | -H | | 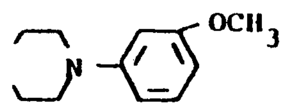 | 3600-2300 $cm^{-1}$ | 1,3 à 2,5 ppm m 4H ; 2,7 à 4,3 ppm m 10H ; 3,8 ppm s 3H ; 4 ppm q 2H ; 6,4 ppm 2H échangeables ; 6,3 à 7,4 ppm 12H. |
| 3b | $-OCH_3$ | $-OCH_3$ | -H | $-CH_3$ | 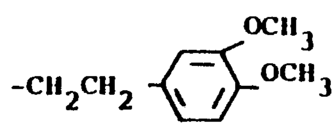 | 3600-2300 $cm^{-1}$ | 1 à 3 ppm m 4H ; 2,4 ppm s 3H ; 2,8 ppm m 6H ; 3,8 à 3,9 ppm m 12H ; 3,9 ppm q 2H ; 6,2 ppm 2H échangeables. 6,8 à 7,9 ppm m 10H. |
| 4b | -Cl | -H | -H | $-CH_3$ | 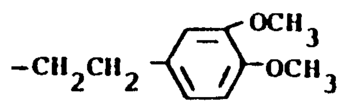 | 3700-2300 $cm^{-1}$ | 0,8 à 1,5 ppm m 2H ; 1,3 à 3 ppm m 11H ; 3 ppm s 6H ; 3 à 4,5 ppm m 2H ; 6 à 7,5 ppm m 11H et 2H échangeables. |
| 5b | $-OCH_3$ | -H | $-CH_3$ | $-CH_3$ | 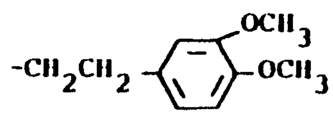 | 3700-2300 $cm^{-1}$ | 1 à 2 ppm m 2H ; 2 à 3 ppm m 14H ; 3,6 à 3,85 ppm m 9H ; 4,2 ppm q 2H ; 6 à 7,5 ppm m 10 H et 2H échangeables. |
| 6b | $-OCH_3$ | -H | -H | $-CH_3$ | 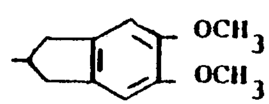 | 3700-2300 $cm^{-1}$ | 1,7 ppm m 4H ; 2,2 ppm s 3H ; 2,3 à 3,7 ppm m 7H ; 3,5 ppm s 3H ; 3,8 ppm s 6H ; 4,2 ppm q 2H ; 6,0 à 7,3 ppm m 10H et 2H échangeables. |

**TABLEAU 2 (suite N° 1) : COMPOSES DE FORMULE GENERALE V**

| EX | $X_1$ | $X_2$ | $X_3$ | $R_1$ | $R_2$ | IR ν(OH) | RMN |
|---|---|---|---|---|---|---|---|
| 7b | $-OCH_3$ | -H | -H | | 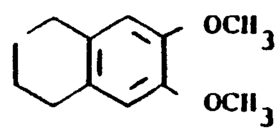 | 3600-2300 $cm^{-1}$ | 1 à 2 ppm m 2H ; 2 à 3 ppm m 8H ; 3,5 ppm s 2H ; 3,6 ppm s 3H ; 3,85 ppm s 6H ; 4,2 ppm q 2H ; 5,2 à 6,2 ppm 2H échangeables ; 6,2 à 6,8 ppm m 5H ; 7,3 ppm m 5H. |
| 8b | $-OCH_3$ | -H | -H | $-CH_3$ | 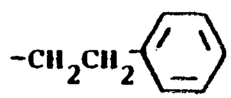 | 3600-2300 $cm^{-1}$ | 1 à 2 ppm m 2H ; 2 à 3,2 ppm m 11H ; 3,6 ppm s 3H ; 4,15 ppm q 2H ; 6,2 à 7,6 ppm m 13H et 2H échangeables. |
| 9b | $-OCH_3$ | -H | -H | | 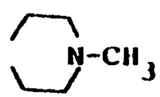 | 3600-2600 $cm^{-1}$ | 1,3 à 2,4 ppm m 4H ; 2,3 ppm s 3H ; 2,5 à 2,8 ppm m 10H ; 3,9 ppm s 3H ; 4,2 ppm q 2H ; 6,5 à 7,3 ppm m 8H et 2H échangeables. |
| 10b | -H | -H | -H | $-C_2H_5$ | $-C_2H_5$ | 3600-2400 $cm^{-1}$ | 0,8 à 2 ppm m 8H ; 2 à 2,6 ppm m 8H ; 3,8 ppm s 3H ; 4,1 ppm q 2H ; 6,4 à 7,5 ppm m 8H et 2H échangeables. |

**TABLEAU 3 : COMPOSES DE FORMULE GENERALE I**

| EX | $X_1$ | $X_2$ | $X_3$ | $R_1$ | $R_2$ | Y | IR $(NH)^+$ | RMN |
|---|---|---|---|---|---|---|---|---|
| 1 | $OCH_3$ | H | H | $CH_3$ | 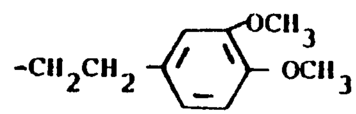 | H | (phosphate) 3500-2000 $cm^{-1}$ | (phosphate) 1,9 à 2,3 ppm m 2H ; 2,4 à 3,1 ppm m 11H ; 3,5 à 3,9 ppm m 9H ; 4,4 ppm s 2H ; 6,6 à 7,2 ppm m 11H ; 9 à 9,6 ppm protons échangeables. |
| 2 | Cl | H | H | | 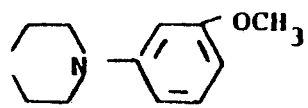 | H | (oxalate) 3000-2000 $cm^{-1}$ | (oxalate) 1,3 à 2,5 ppm m 4H ; 2,7 à 3,5 ppm m 10H ; 3,7 ppm s 3H ; 4,5 ppm s 2H ; 6,3 à 7,5 ppm m 11H ; 9,8 ppm 2H échangeables. |
| 3 | $OCH_3$ | $OCH_3$ | H | $CH_3$ | 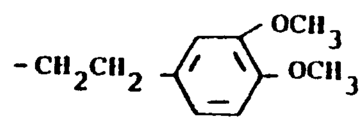 | H | (phosphate) 3500-2000 $cm^{-1}$ | (base) 1 à 2 ppm m 2H ; 1,8 à 2,8 ppm m 11H ; 3,8 à 3,9 ppm m 12H ; 4,5 ppm s 2H ; 6,5 à 7 ppm m 5H ; 7,3 ppm m 5H. |
| 4 | Cl | H | H | $CH_3$ | 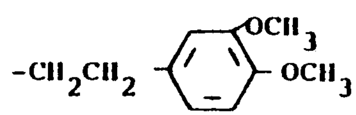 | H | (chlorhydrate) 3500-2000 $cm^{-1}$ | (chlorhydrate) 1 à 2 ppm m 2H ; 2 à 3,2 ppm m 11H ; 3,9 ppm s 6H ; 4,55 ppm s 2H ; 6,7 à 7,5 ppm m 11H. |

TABLEAU 3 (suite N° 1) : COMPOSES DE FORMULE GENERALE I

| EX | $X_1$ | $X_2$ | $X_3$ | $R_1$ | $R_2$ | Y | IR $(NH)^+$ | RMN |
|---|---|---|---|---|---|---|---|---|
| 5 | $OCH_3$ | H | $CH_3$ | $CH_3$ | 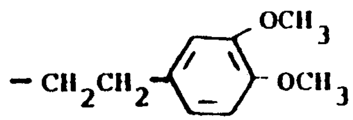 | H | (phosphate) 3700-2000 $cm^{-1}$ | (phosphate) 1 à 2 ppm m 2H ; 1,3 à 2,8 ppm m 14H ; 3,85 ppm s 3,95 ppm s 6H ; ; 4,5 ppm s 2H ; 6,5 à 7,5 ppm m 10H. |
| 6 | $OCH_3$ | H | H | $CH_3$ | 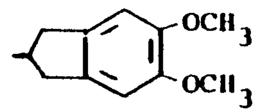 | H | (phosphate) 3600-2000 $cm^{-1}$ | (phosphate) 2 à 3 ppm m 14H ; 3,5 ppm s 6H ; 3,6 ppm s 6H ; 4,3 ppm s 2H ; 6,5 à 7,3 ppm m 10H. |
| 7 | $OCH_3$ | H | H | | 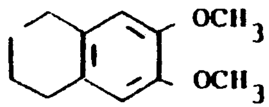 | H | (phosphate) 3600-2000 $cm^{-1}$ | (phosphate) 1,3 à 3,5 ppm m 10H ; 3,5 à 4 ppm m 11H ; 4,5 s 2H ; 6 à 7,5 ppm m 10H ; 9,7 ppm H échangeable. |
| 8 | $OCH_3$ | H | H | $CH_3$ | 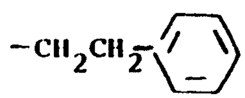 | H | (phosphate) 37000-2000 $cm^{-1}$ | (phosphate) 1 à 2,5 ppm m 2H ; 2,5 à 3,5 ppm m 11H ; 3,6 ppm s 3H ; 4,5 ppm s 2H ; 6,5 à 7,6 ppm m 13H ; 9,4 ppm H échangeable. |
| 9 | $OCH_3$ | H | H | | 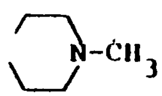 | H | (chlorhydrate) 3600-2150 $cm^{-1}$ | (chlorhydrate) 1,5 à 2,5 ppm m 4H ; 3 ppm s 3H ; 3,8 ppm 2H échangeables ; 3,8 ppm t 10H ; 3,9 ppm s 3H ; 4,6 ppm s 2H ; 6,9 à 7,5 ppm m 8H. |

TABLEAU 3 (suite N° 2) : COMPOSES DE FORMULE GENERALE I

| EX | $X_1$ | $X_2$ | $X_3$ | $R_1$ | $R_2$ | Y | IR $(NH)^+$ | RMN |
|---|---|---|---|---|---|---|---|---|
| 10 | $OCH_3$ | H | H | $C_2H_5$ | $-C_2H_5$ | H | (chlorhydrate) 2500 $cm^{-1}$ | (chlorhydrate) 1,3 ppm t 6H ; 2,1 ppm m 4H ; 3 ppm m 6H ; 3,8 ppm s 3H ; 4,6 ppm s 2H ; 6,8 ppm 3H ; 7,4 ppm m 5H ; 11 ppm H échangeable. |
| 11 | $OCH_3$ | H | H | $CH_3$ | 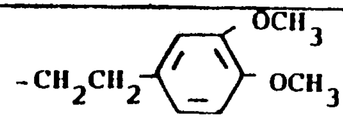 | OH | (base) 3500-3400 $cm^{-1}$ | (base) 1,4 ppm m 2H ; 2,2 à 2,8 ppm m 11H ; 3,7 à 4 ppm m 9H ; 5,8 ppm s 1H ; 6,4 à 6,9 ppm m 6H ; 7,3 ppm m 5H et H échangeable. |
| 12 | $OCH_3$ | $OCH_3$ | H | $CH_3$ | 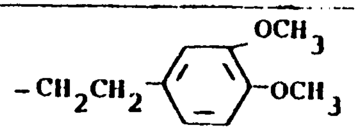 | OH | (base) 3500-3400 $cm^{-1}$ | (base) 1,4 ppm m 2H ; 2,2 à 2,8 ppm m 11H ; 3,4 à 4 ppm m 12H ; 5,8 ppm s 1H ; 6,4 à 6,9 ppm m 5H ; 7,3 ppm m 5H et H échangeable. |
| 13 | H | H | H | $CH_3$ | 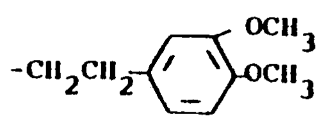 | H | (phosphate) 3700-2200 $cm^{-1}$ | (phosphate) 1 à 2,5 ppm m 2H ; 2,5 à 3,5 ppm m 11H ; 3,7 ppm s 6H ; 4,5 ppm m 2H ; 6,5 à 7,7 ppm m 12H ; 10,1 ppm H échangeable. |

TABLEAU 3 (suite N° 3) : COMPOSES DE FORMULE GENERALE I

| EX | $X_1$ | $X_2$ | $X_3$ | $R_1$ | $R_2$ | Y | IR $(NH)^+$ | RMN |
|---|---|---|---|---|---|---|---|---|
| 14 | H | H | H | | 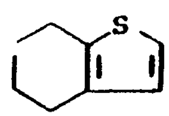 | H | (citrate) 3600-2000 $cm^{-1}$ | (citrate) 1 à 2,5 ppm m 6H ; 2,5 à 3,5 ppm m 8H ; 3,55 ppm s 2H ; 4,55 ppm s 2H ; 6,5 à 7,6 ppm m 11H ; 8,2 ppm 4H échangeables. |
| 15 | H | H | H | | 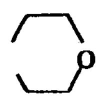 | H | (chlorhydrate) 2100-2700 $cm^{-1}$ | (base) 1 à 1,8 ppm m 10H ; 3,5 à 3,8 ppm m 4H ; 4,5 ppm s 2H ; 6,7 à 7,6 ppm m 9H. |
| 16 | H | H | H | | 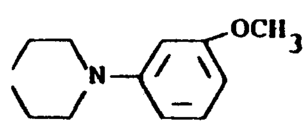 | H | (chlorhydrate) 2100-2700 $cm^{-1}$ | (chlorhydrate) 1,5 à 2,5 ppm m 4H ; 2,7 à 4,3 ppm m 10H ; 3,8 ppm s 3H ; 4,5 ppm s 2H ; 6,5 à 7,5 ppm m 13H ; 13 ppm H échangeable. |

**Revendications** pour les Etats Contractants: BE, CH, DE, FR, GB, IT, LI, LU, NL, SE

1. Composés de formule générale I:

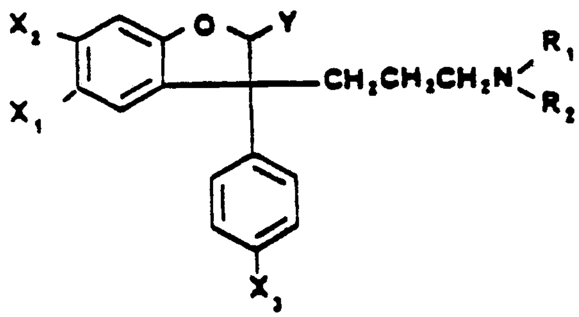

(I)

dans laquelle:

- $X_1$ et $X_2$, identiques ou différents représentent chacun un atome d'hydrogène ou d'halogène ou un radical alcoxy renfermant de 1 à 4 atomes de carbone, ou forment ensemble un radical méthylènedioxy,
- $X_3$ représente un atome d'hydrogène ou un radical alkyle de 1 à 4 atomes de carbone,
- Y représente un atome d'hydrogène ou un radical hydroxy,
- $R_1$ et $R_2$ identiques ou différents représentent chacun un radical alkyle de 1 à 4 atomes de carbone, à condition toutefois que, quand Y représente un atome d'hydrogène, $X_1$ ou $X_2$ ou $X_1$ et $X_2$ simultanément représentent un radical alcoxy renfermant de 1 à 4 atomes de carbone,
- ou bien

$R_1$ est un méthyle et,

$R_2$ représente un groupement phénylalkyle renfermant de 7 à 9 atomes de carbone ou un groupe indanyle-2, éventuellement substitués sur le cycle aromatique par un ou deux radicaux alcoxy renfermant de 1 à 4 atomes de carbone, à condition toutefois que $R_2$ ne représente jamais un phénethyle quand $X_1$ et $X_2$ représentent chacun un atome d'hydrogène ou d'halogène et Y représente un atome d'hydrogène,

ou bien

$R_1$ et $R_2$ forment ensemble, avec l'atome d'azote auxquels ils sont attachés,

un radical morpholinyle-4, un radical tétrahydro-1,2,6,7 thiéno(2,3-c) pyridinyle-1, un radical tetrahydroisoquinolyle-2 éventuellement substitué par un ou deux radicaux alcoxy de 1 à 4 atomes de carbone, un groupe alkyle-4 (de 1 à 4 atomes de carbone) piperazinyle-1, ou un groupe phényl-4 pipérazinyle-1 éventuellement substitué au niveau du cycle aromatique par 1 ou 2 radicaux alcoxy renfermant de 1 à 4 atomes de carbone,

sous forme racémique ou d'isomères optiques et leurs sels d'addition à un acide minéral ou organique pharmaceutiquement acceptable.

2. Le méthoxy-5 phényl-3 [(N-(diméthoxy-3,4 phénéthyl) N-méthylamino)-3 propyl]-3 dihydro-2,3 benzofuranne, composé selon la formule générale I de la revendication 1, ses isomères optiques et ses sels avec un acide pharmaceutiquement compatible.

3. Le phényl-3 [(N-diméthoxy-3,4 phénéthyl)N-méthylamino)-3 propyl]-3 dihydro-2,3 benzofuranne, composé selon la formule générale I de la revendication 1, ses isomères optiques et ses sels avec un acide pharmaceutiquement compatible.

4. Procédé de préparation des composés de formule générale I, de la revendication 1, caractérisé en ce que l'on soumet une aryl-3 (chloro-3 propyl) -3 benzofuranne-2 de formule générale II:

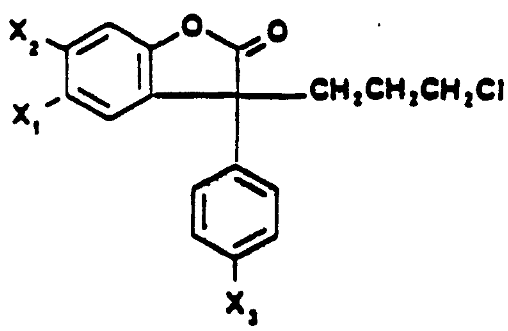

(II)

dans laquelle la définition des substituants $X_1$, $X_2$, $X_3$ est identique à celle donnée pour la formule générale I de la revendication 1,

<u>soit</u>: à l'action d'une amine secondaire de formule générale III:

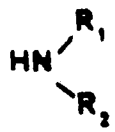

(III)

dans laquelle la définition de $R_1$ et $R_2$ demeure celle indiquée pour la formule générale I de la revendication 1, dans un solvant organique polaire à une température comprise entre 50° et 90°C et en présence de sels minéraux, pour former un dérivé de formule générale IV:

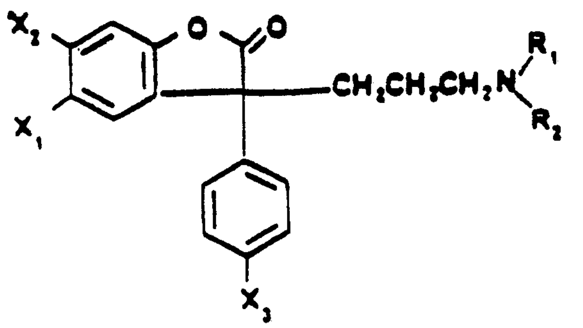

(IV)

dans laquelle $X_1$, $X_2$, $X_3$, $R_1$, $R_2$ ont les significations précédemment définies pour la formule générale I de la revendication 1,

- que l'on réduit partiellement dans un solvant organique aprotique, à une température comprise entre -50°C et -80°C et en présence d'hydrure de diisobutylaluminium en un dérivé de formule générale I':

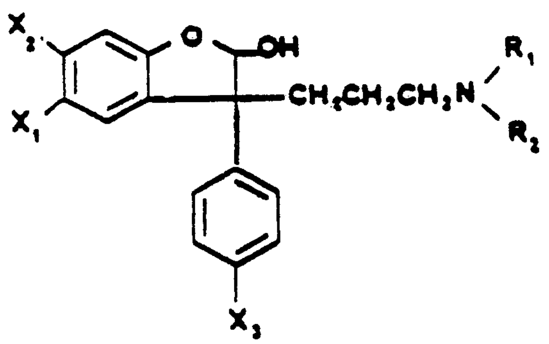

(I')

dans laquelle $X_1$, $X_2$, $X_3$, $R_1$ et $R_2$ sont tels que précédemment définis pour la formule générale I de la revendication 1,

que l'on sépare ensuite si l'on désire, en ses isomères optiques, ou transforme en un sel d'addition avec un acide pharmaceutiquement acceptable,

- ou que l'on ouvre par réduction à l'aide d'hydrures métalliques dans un solvant organique inerte à une température comprise entre 15°C et 40°C et ensuite hydrolyse en un diol de formule générale V:

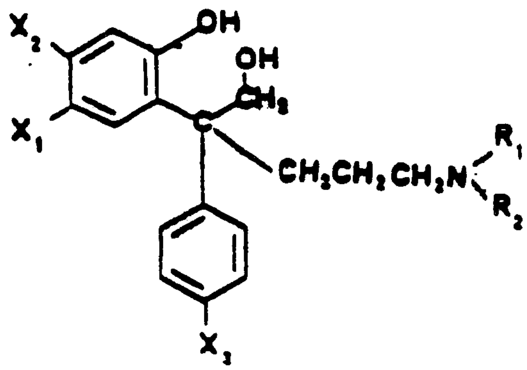

(V)

dans laquelle $X_1$, $X_2$, $X_3$, $R_1$, $R_2$ ont les significations précédemment définies pour la formule générale I de la revendication 1, que l'on cyclise par l'action de butyllithium et du chlorure de paratoluènesulfonyle en un composé de formule générale I":

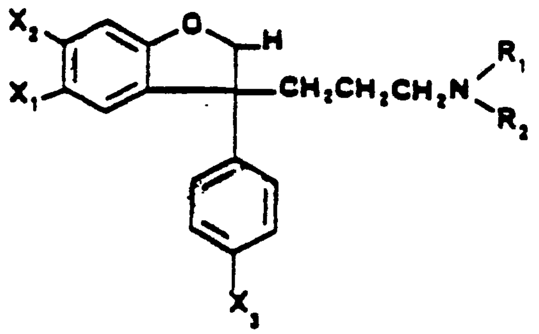

(I")

dans laquelle la signification de $X_1$, $X_2$, $X_3$, $R_1$ et $R_2$ est identique à celle de la formule générale I, de la revendication 1, que l'on sépare ensuite, si l'on désire, en ses isomères optiques ou salifie avec un acide pharmaceutiquement acceptable,

soit à l'action d'un hydrure métallique dans un solvant organique et à une température comprise entre 15°C et 40°C pour obtenir, après hydrolyse, un diol de formule générale VI:

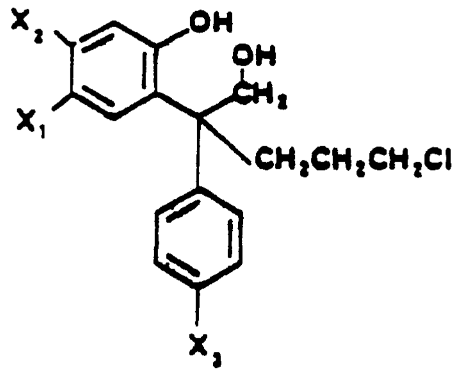

(VI)

dans laquelle la définition de $X_1$, $X_2$, $X_3$ demeure celle indiquée précédemment dans la formule générale I, de la revendication 1,

que l'on cyclise par l'action de butyllithium et du chlorure de paratoluènesulfonyle, pour obtenir un composé de formule générale VII:

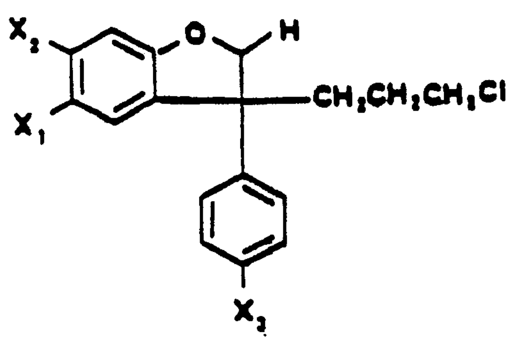

(VII)

dans laquelle la définition de $X_1$, $X_2$, $X_3$ a les significations précédemment définies pour la formule générale I de la revendication 1,

et que l'on condense dans un solvant polaire et à une température comprise entre 50°C et 90°C et en présence de sels minéraux avec une amine de formule générale III pour obtenir un composé de formule générale I''

et séparer ensuite si l'on désire ses isomères optiques ou ou transforme en un sel d'addition avec un acide pharmaceutiquement acceptable.

5. Compositions pharmaceutiques contenant comme principe actif un composé selon l'une quelconque des revendications 1 à 3, en association ou en mélange avec un ou plusieurs excipients ou un véhicule inerte non toxique, pharmaceutiquement acceptable.

6. Une composition pharmaceutique selon la revendication 5 utilisable dans le traitement des maladies nécessitant des modulateurs de mouvements transmembranaires et intracellulaires du calcium.

**Revendications** pour l'Etat Contractant: AT

1. Procédé de préparation de dérivés de formule générale I:

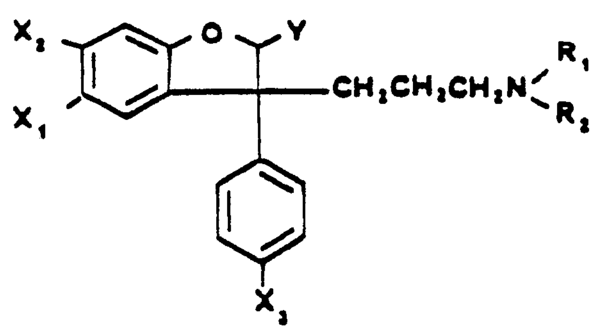

(I)

dans laquelle:

- $X_1$ et $X_2$, identiques ou différents représentent chacun un atome d'hydrogène ou d'halogène, un radical alcoxy renfermant de 1 à 4 atomes de carbone, ou forment ensemble un radical méthylènedioxy,
- $X_3$ représente un atome d'hydrogène ou un radical alkyle de 1 à 4 atomes de carbone,
- Y représente un atome d'hydrogène ou un radical hydroxy,
- $R_1$ et $R_2$ identiques ou différents représentent chacun un radical alkyle de 1 à 4 atomes de carbone, à condition toutefois que quand Y représente un atome d'hydrogène, $X_1$ ou $X_2$ ou $X_1$ et $X_2$ simultanément représentent un radical alcoxy renfermant de 1 à 4 atomes de carbone,
- ou bien

$R_1$ est un méthyle et,

$R_2$ représente un groupement phénylalkyle renfermant de 7 à 9 atomes de carbone ou un groupe indanyle-2, éventuellement substitués sur le cycle aromatique par un ou deux radicaux alcoxy renfermant de 1 à 4 atomes de carbone, à condition toutefois que $R_2$ ne représente jamais un phénethyle quand $X_1$ et $X_2$ représentent chacun un atome d'hydrogène ou d'halogène et Y représente un atome d'hydrogène,

- ou bien

$R_1$ et $R_2$ forment ensemble, avec l'atome d'azote auxquels ils sont attachés,

un radical morpholinyle-4, un radical tétrahydro-1,2,6,7 thiéno(2,3-c) pyridinyle-1, un radical tétrahydroisoquinolyle-2 éventuellement substitué par un ou deux radicaux alcoxy de 1 à 4 atomes de carbone, un groupe alkyle-4 (de 1 à 4 atomes de carbone) piperazinyle-1, ou un groupe phényl-4 piperazinyle-1 éventuellement substitué au niveau du cycle aromatique par 1 ou 2 radicaux alcoxy renfermant de 1 à 4 atomes de carbone,

sous forme racémique ou d'isomères optiques et leurs sels d'addition à un acide minéral ou organique pharmaceutiquement acceptable,

caractérisé en ce que l'on soumet une aryl-3 (chloro-3 propyl) -3 benzofuranne-2 de formule générale II:

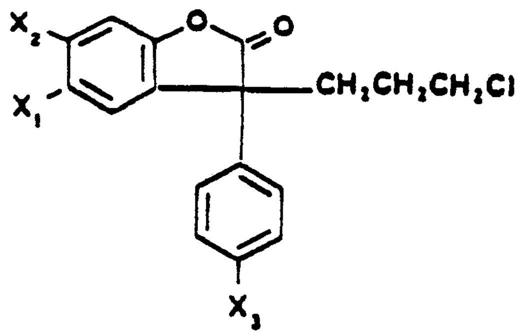

(II)

dans laquelle la définition des substituants $X_1$, $X_2$, $X_3$ demeure identique à celle donnée pour la formule générale I,

<u>soit</u>: à l'action d'une amine secondaire de formule générale III:

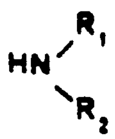

(III)

dans laquelle la définition de $R_1$ et $R_2$ demeure celle indiquée précédemment, pour la formule générale I, dans un solvant organique polaire à une température comprise entre 50° et 90°C et en présence de sels minéraux, pour former un dérivé de formule générale IV:

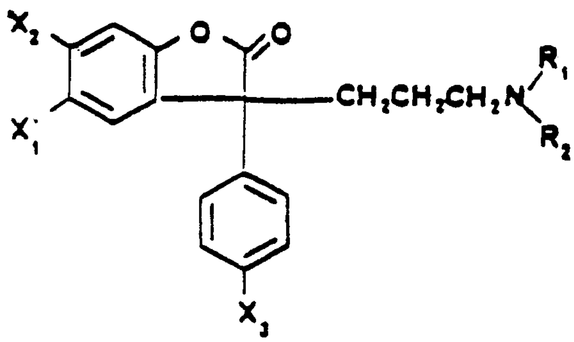

(IV)

dans laquelle $X_1$, $X_2$, $X_3$, $R_1$, $R_2$ ont les significations précédemment définies pour la formule I,

- que l'on réduit partiellement dans un solvant organique aprotique, à une température comprise entre -50°C et -80°C et en présence d'hydrure de diisobutylaluminium en un dérivé de formule générale I':

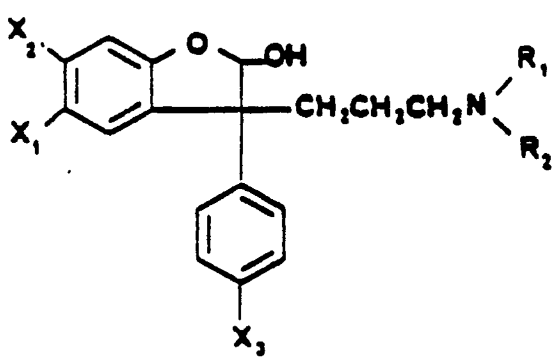

(I')

dans laquelle $X_1$, $X_2$, $X_3$, $R_1$ et $R_2$ sont tels que précédemment définis pour la formule générale I,

que l'on sépare ensuite, si l'on désire, en ses isomères optiques ou salifie avec un acide

pharmaceutiquement acceptable,

- ou que l'on ouvre par réduction à l'aide d'hydrures métalliques dans un solvant organique inerte à une température comprise entre 15° C et 40° C et ensuite hydrolyse en un diol de formule générale V:

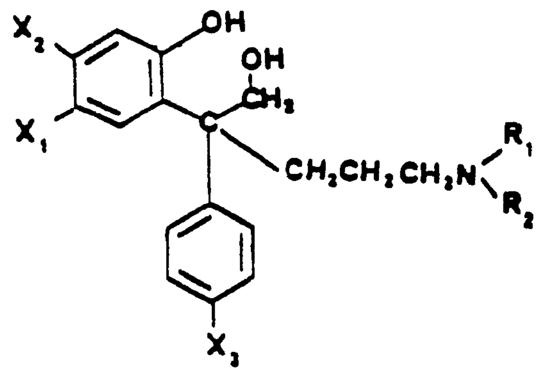

(V)

dans laquelle $X_1$, $X_2$, $X_3$, $R_1$, $R_2$ ont les significations précédemment définies pour la formule générale I, que l'on cyclise par l'action du butyllithium et du chlorure de paratoluènesulfonyle en un composé de formule générale I'':

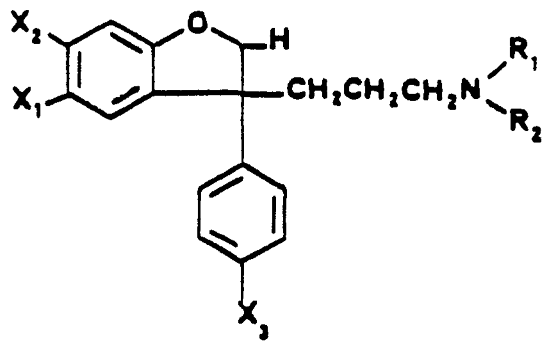

(I'')

dans laquelle la signification de $X_1$, $X_2$, $X_3$, $R_1$ et $R_2$ est identique à celle de la formule générale I,

que l'on sépare ensuite, si l'on désire, en ses isomères optiques ou salifie avec un acide pharmaceutiquement acceptable,

soit à l'action d'un hydrure métallique dans un solvant organique et à une température comprise entre 15° C et 40° C pour obtenir, après hydrolyse, un diol de formule générale VI:

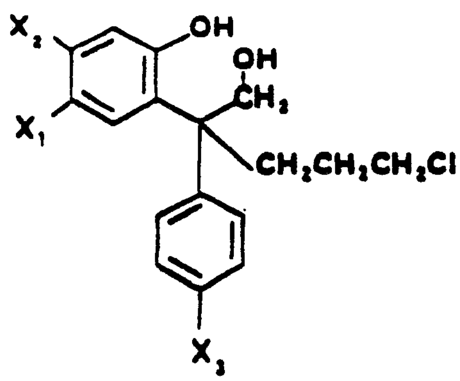

(VI)

dans laquelle la définition de $X_1$, $X_2$, $X_3$ demeure celle indiquée précédemment dans la formule générale I,

que l'on cyclise par l'action de butyllithium et du chlorure de paratoluènesulfonyle, pour obtenir un composé de formule générale VII:

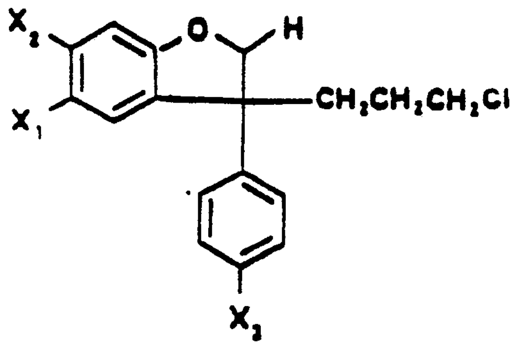

(VII)

dans laquelle la définition de $X_1$, $X_2$, $X_3$ a les significations précédemment définies pour la formule générale I

et que l'on condense dans un solvant polaire et à une température comprise entre 50°C et 90°C et en présence de sels minéraux avec une amine de formule générale III pour obtenir un composé de formule générale I''

et séparer ensuite si l'on désire ses isomères optiques ou transforme en un sel d'addition avec un acide pharmaceutiquement acceptable.

**Patentansprüche** für die Vertragsstaaten: BE, CH, DE, FR, GB, IT, LI, LU, NL, SE

1. Verbindungen der allgemeinen Formel I

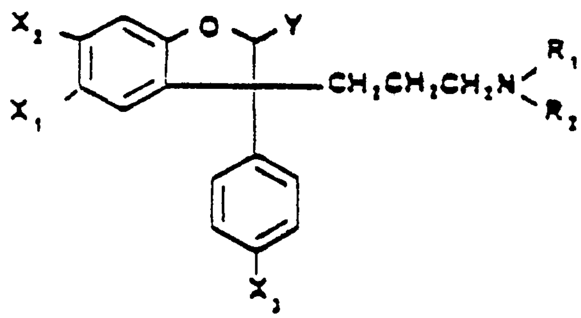

(I)

in der

- $X_1$ und $X_2$, die gleichartig oder verschieden sein können, Wasserstoffatome, Halogenatome, Alkoxygruppen mit 1 bis 4 Kohlenstoffatomen oder gemeinsam eine Methylendioxygruppe,
- $X_3$ ein Wasserstoffatom oder eine Alkylgruppe mit 1 bis 4 Kohlenstoffatomen,
- Y ein Wasserstoffatom oder eine Hydroxygruppe,
- $R_1$ und $R_2$, die gleichartig oder verschieden sein können, Alkylgruppen mit 1 bis 4 Kohlenstoffatomen, mit der Maßgabe, daß wenn Y ein Wasserstoffatom darstellt, $X_1$ oder $X_2$ oder $X_1$ und $X_2$ gleichzeitig eine Alkoxygruppe mit 1 bis 4 Kohlenstoffatomen darstellen,

oder

$R_1$ eine Methylgruppe und

$R_2$ eine Phenylalkylgruppe mit 7 bis 9 Kohlenstoffatomen oder eine Indan-2-ylgruppe, die gegebenenfalls am aromatischen Ring durch eine oder zwei Alkoxygruppen mit 1 bis 4 Kohlenstoffatomen substituiert ist,

mit der Maßgabe, daß $R_2$ keine Phenethylgruppe darstellt, wenn $X_1$ und $X_2$ jeweils Wasserstoffatome oder Halogenatome und Y ein Wasserstoffatom darstellen,

oder

$R_1$ und $R_2$ gemeinsam mit dem Stickstoffatom, an das sie gebunden sind, einen Morpholin-4-yl-Rest, einen 1,2,6,7-Tetrahydro-thieno-(2,3-c)-pyridin-1-yl-Rest, einen Tetrahydroisochinol-2-yl-Rest, der gegebenenfalls durch eine oder zwei Alkoxygruppen mit 1 bis 4 Kohlenstoffatomen substituiert ist, eine 4-Alkyl-piperazin-1-yl-Gruppe (mit 1 bis 4 Kohlenstoffatomen im Alkylrest) oder eine 4-Phenyl-piperazin-1-yl-Gruppe, die gegebenenfalls am aromatischen Ring durch eine oder zwei Alkoxygruppen mit 1 bis 4 Kohlenstoffatomen substituiert ist,

bedeuten, in Form des Racemats oder der optischen Isomeren sowie deren Additionssalze mit einer anorganischen oder organischen pharmazeutisch annehmbaren Säure.

2. 5-Methoxy-3-phenyl-3-[3-(N-(3,4-dimethoxy-phenethyl)-N-methylamino)-propyl]-2,3-dihydro-benzofuran als Verbindung der allgemeinen Formel I von Anspruch 1, die optischen Isomeren und die Salze dieser Verbindung mit einer pharmazeutisch verträglichen Säure.

3. 3-Phenyl-3-[3-(N-(3,4-dimethoxy-phenethyl)-N-methylamino)-propyl]-2,3-dihydrobezofuran als Verbindung der allgemeinen Formel I von Anspruch 1, die optischen Isomeren und die Salze dieser Verbindung mit einer pharmazeutisch annehmbaren Säure.

4. Verfahren zur Herstellung der Verbindungen der allgemeinen Formel I von Anspruch 1, dadurch gekennzeichnet, daß man ein 3-Aryl-3-(3-chlor-propyl)-benzofuran-2 der allgemeinen Formel II:

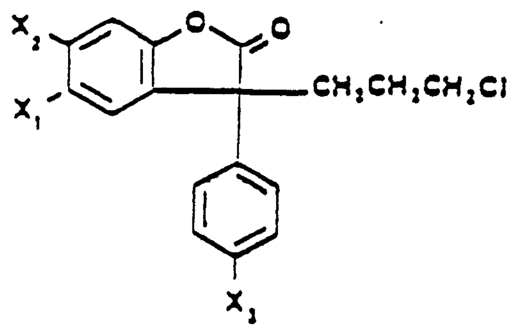

(II)

in der die Definition der Substituenten $X_1$, $X_2$ und $X_3$ identisch ist mit der für die allgemeine Formel I von Anspruch 1 angegebenen,

entweder: mit einem sekundären Amin der allgemeinen Formel III

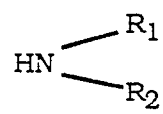

in der die Definition für $R_1$ und $R_2$ der in Anspruch 1 für die allgemeine Formel I angegebenen entspricht, in einem polaren organischen Lösungsmittel bei einer Temperatur zwischen 50 und 90° in Gegenwart von Mineralsalzen zur Bildung eines Derivats der allgemeinen Formel IV umsetzt:

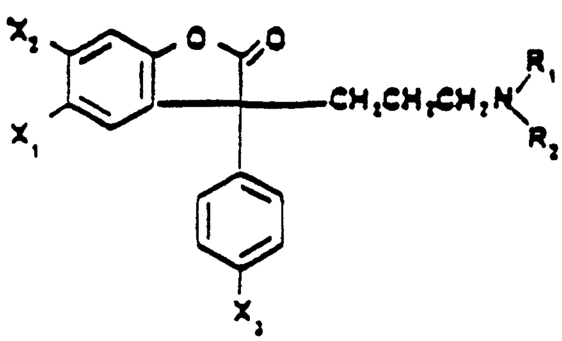

(IV)

in der $X_1$, $X_2$, $X_3$, $R_1$ und $R_2$ die oben bezüglich der allgemeinen Formel I von Anspruch 1 angegebenen Bedeutungen besitzen,

- welche man in einem aprotischen organischen Lösungsmittel bei einer Temperatur zwischen -50°C und -80°C in Gegenwart von Diisobutylaluminiumhydrid teilweise reduziert zu einem Derivat der allgemeinen Formel I'

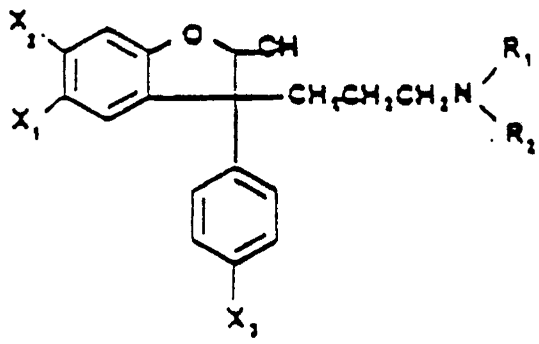

(I')

in der $X_1$, $X_2$, $X_3$, $R_1$ und $R_2$ die oben bezüglich der allgemeinen Formel I von Anspruch 1 angegebenen Bedeutungen besitzen,

- welche man anschließend gewünschtenfalls in die optischen Isomeren auftrennt oder mit einer pharmazeutischen annehmbaren Säure in ein Additionsalz überfürt,
- oder durch Reduktion mit einem Metallhydrid in einem inerten organischen Lösungsmittel bei einer Temperatur zwischen 15° C und 40° C und anschließende Hydrolyse in ein Diol der allgemeinen Formel V

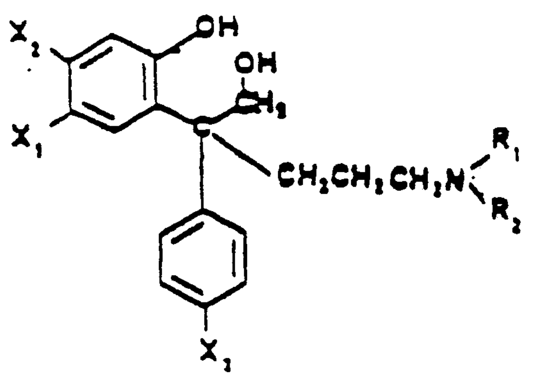

(V)

umwandelt, worin $X_1$, $X_2$, $X_3$, $R_1$ und $R_2$ die oben bezüglich der allgemeinen Formel I von Anspruch 1 angegebenen Bedeutungen besitzen,

welches man durch Umsetzung mit Butyllithium und p-Toluolsulfonylchlorid zu einer Verbindung der allgemeinen Formel I''

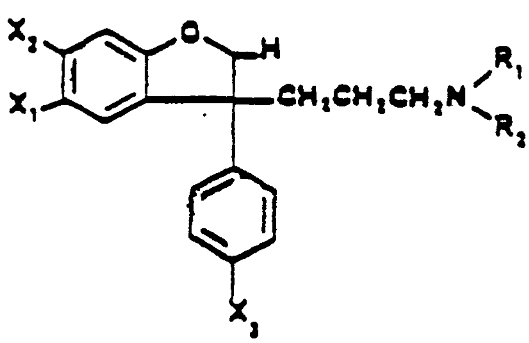

(I")

cyclisiert, worin die Bedeutungen von $X_1$, $X_2$, $X_3$, $R_1$ und $R_2$ identisch sind mit den Anspruch 1 bezüglich der allgemeinen Formel I angegebenen,

welche man gewünschtenfalls in die optischen Isomeren aufspaltet oder mit einer pharmazeutisch annehmbaren Säure in ein Salz überführt,

oder mit einem Metallhydrid in einem organischen Lösungsmittel bei einer Temperatur zwischen 15°C und 40°C umsetzt um nach Durchführung einer Hydrolyse ein Diol der allgemeinen Formel VI

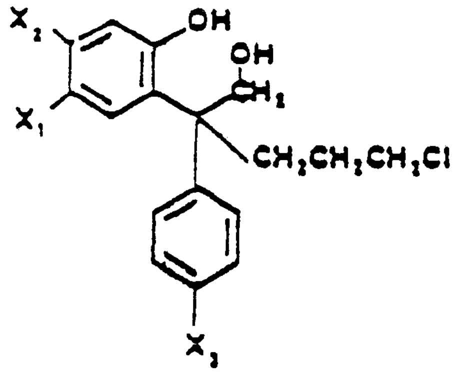

(VI)

zu erhalten, in der die Definition von $X_1$, $X_2$ und $X_3$ identisch derjenigen ist, die in Anspruch 1 bezüglich der allgemeinen Formel I angegeben worden ist,

welche man durch Umsetzung mit Butyllithium und p-Toluolsulfonylchlorid zu einer Verbindung der allgemeinen Formel VII

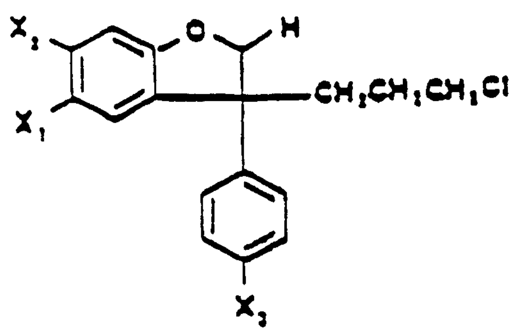

(VII)

cyclisiert, in der die Definition von $X_1$, $X_2$ und $X_3$ derjenigen entspricht, die in Anspruch 1 bezüglich der allgemeinen Formel I angegeben ist,

die man in einem polaren Lösungsmittel bei einer Temperatur zwischen 50°C und 90°C in Gegenwart von Mineralsalzen mit einem Amin der allgemeinen Formel III zur Bildung einer Verbindung der allgemeinen Formel I'' kondensiert und anschließend gewünschtenfalls in die optischen Isomeren aufspaltet oder mit einer pharmzeutisch annehmbaren Säure in ein Additionssalz umwandelt.

5. Pharmazeutische Zubereitungen enthaltend als Wirkstoff eine Verbindung nach einem der Ansprüche 1 bis 3 in Kombination oder in Mischung mit einem oder mehreren inerten oder nicht toxischen pharmazeutisch annehmbaren Trägermaterialien oder Hilfsstoffen.

6. Pharmazeutische Zubereitung nach Anspruch 5, dadurch gekennzeichnet, daß sie in einer zur Behandlung von Krankheiten, die Modulatoren für transmembrane und intrazelluläre Bewegungen des Calciums erforderlich machen, geeigneten Form vorliegt.

**Patentansprüche** für den Vertragsstaat: AT

1. Verfahren zur Herstellung von Derivaten der allgemeinen Formel I

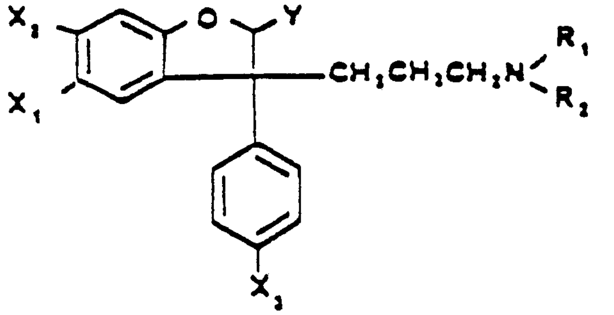

(I)

in der

- $X_1$ und $X_2$, die gleichartig oder verschieden sein können, Wasserstoffatome, Halogenatome, Alkoxygruppen mit 1 bis 4 Kohlenstoffatomen oder gemeinsam eine Methylendioxygruppe,
- $X_3$ ein Wasserstoffatom oder eine Alkylgruppe mit 1 bis 4 Kohlenstoffatomen,
- Y ein Wasserstoffatom oder eine Hydroxygruppe,
- $R_1$ und $R_2$, die gleichartig oder verschieden sein können, Alkylgruppen mit 1 bis 4 Kohlenstoffatomen, mit der Maßgabe, daß wenn Y ein Wasserstoffatom darstellt, $X_1$ oder $X_2$ oder $X_1$ und X2 gleichzeitig eine Alkoxygruppe mit 1 bis 4 Kohlenstoffatomen darstellen,

oder

$R_1$ eine Methylgruppe und

$R_2$ eine Phenylalkylgruppe mit 7 bis 9 Kohlenstoffatomen oder eine Indan-2-ylgruppe, die gegebenenfalls am aromatischen Ring durch eine oder zwei Alkoxygruppen mit 1 bis 4 Kohlenstoffatomen substituiert ist,

mit der Maßgabe, daß $R_2$ keine Phenethylgruppe darstellt, wenn $X_1$ und $X_2$ jeweils Wasserstoffatome oder Halogenatome und Y ein Wasserstoffatom darstellen,

oder

$R_1$ und $R_2$ gemeinsam mit dem Stickstoffatom, an das sie gebunden sind, einen Morpholin-4-yl-Rest, einen 1,2,6,7-Tetrahydro-thieno-(2,3-c) -pyridin-1-yl-Rest, einen Tetrahydroisochinol-2-yl-Rest, der gegebenenfalls durch eine oder zwei Alkoxygruppen mit 1 bis 4 Kohlenstoffatomen substituiert ist, eine 4-Alkyl-piperazin-1-yl-Gruppe (mit 1 bis 4 Kohlenstoffatomen im Alkylrest) oder eine 4-Phenyl-piperazin-1-yl-Gruppe, die gegebenenfalls am aromatischen Ring durch eine oder zwei Alkoxygruppen mit 1 bis 4 Kohlenstoffatomen substituiert ist,

bedeuten,

in Form des Racemats oder der optischen Isomeren sowie ihrer Additionssalze mit einer pharmazeutisch annehmbaren anorganischen oder organischen Säure, dadurch gekennzeichnet, daß man ein 3-Aryl-3-(3-chlor-propyl)-bezofuran-2 der allgemeinen Formel II:

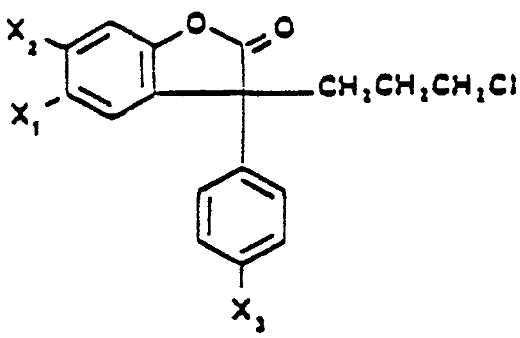

(II)

wobei die Definition der Substituenten $X_1$, $X_2$ und $X_3$ identisch der für die allgemeine Formel I angegebenen ist,

entweder: mit einem sekundären Amin der allgemeinen Formel III

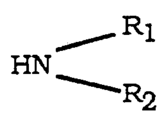

wobei die Definition für $R_1$ und $R_2$ der für die allgemeine Formel I angegebenen entspricht, in einem polaren organischen Lösungsmittel bei einer Temperatur zwischen 50 und 90°C in Gegenwart von Mineralsalzen zur Bildung eines Derivats der allgemeinen Formel IV umsetzt

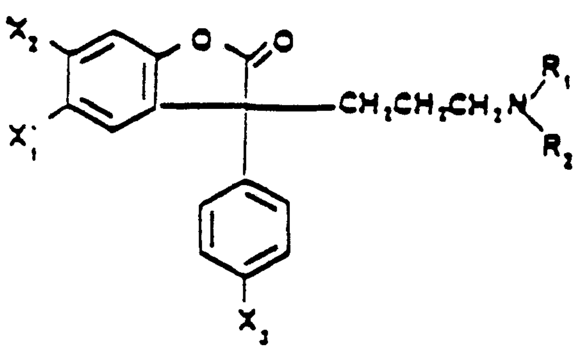

(IV)

in der $X_1$, $X_2$, $X_3$, $R_1$ und $R_2$ die oben bezüglich der allgemeinen Formel I von Anspruch 1 angegebenen Bedeutungen besitzen,

- welche man in einem aprotischen organischen Lösungsmittel bei einer Temperatur zwischen -50°C und -80°C in Gegenwart von Diisobutylaluminiumhydrid teilweise reduziert zu einem Derivat der allgemeinen Formel I'

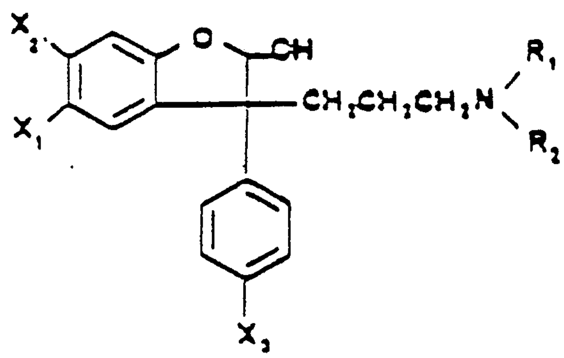

(I')

in der $X_1$, $X_2$, $X_3$, $R_1$ und $R_2$ die oben bezüglich der allgemeinen Formel I angegebenen Bedeutungen besitzen,

- welche man anschließend gewünschtenfalls in die optischen Isomeren auftrennt oder mit einer pharmazeutischen annehmbaren Säure in ein Additionsalz überführt,

- oder durch Reduktion mit einem Metallhydrid in einem inerten organischen Lösungsmittel bei einer Temperatur zwischen 15°C und 40°C und anschließende Hydrolyse in ein Diol der allgemeinen Formel V

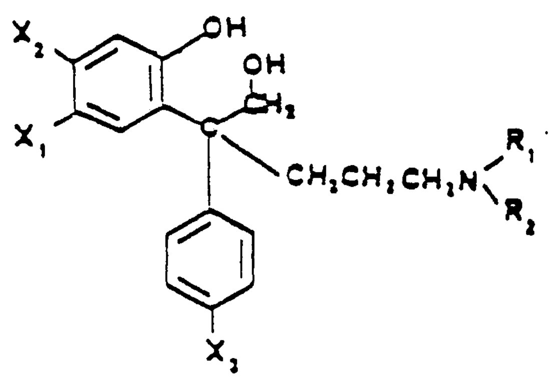

(V)

umwandelt, worin $X_1$, $X_2$, $X_3$, $R_1$ und $R_2$ die oben bezüglich der allgemeinen Formel I angegebenen Bedeutungen besitzen,

welches man durch Umsetzung mit Butyllithium und p-Toluolsulfonylchlorid zu einer Verbindung der allgemeinen Formel I''

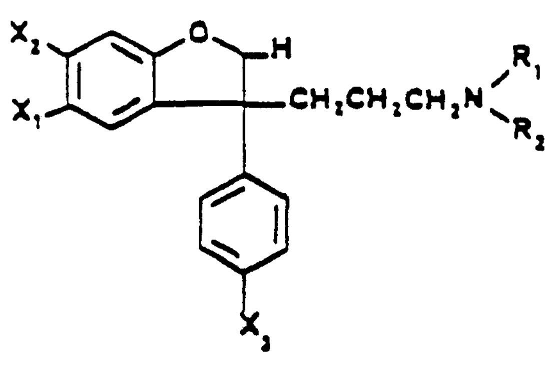

(I'')

cyclisiert, worin die Bedeutung von $X_1$, $X_2$, $X_3$, $R_1$ und $R_2$ identisch ist mit der bezüglich der allgemeinen Formel I angegebenen,

welche man gewünschtenfalls in die optischen Isomeren aufspaltet oder mit einer pharmazeutisch annehmbaren Säure in ein Salz überführt,

oder mit einem Metallhydrid in einem organischen Lösungsmittel bei einer Temperatur zwischen 15°C und 40°C umsetzt um nach Durchführung einer Hydrolyse ein Diol der allgemeinen Formel VI

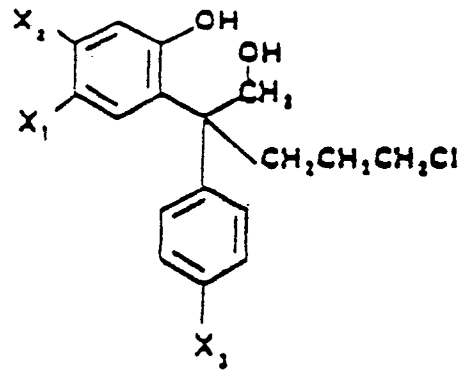

(VI)

zu erhalten, in der die Definition von $X_1$, $X_2$ und $X_3$ identisch derjenigen ist, die bezüglich der allgemeinen Formel I angegeben worden ist,

welche man durch Umsetzung mit Butyllithium und p-Toluolsulfonylchlorid zu einer Verbindung der allgemeinen Formel VII

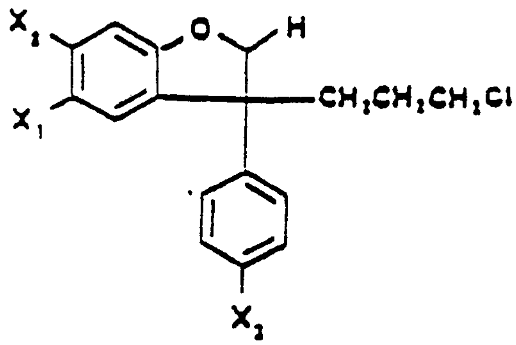

(VII)

cyclisiert, in der die Definition von $X_1$, $X_2$ und $X_3$ derjenigen entspricht, die bezüglich der allgemeinen Formel I angegeben ist,

die man in einem polaren Lösungsmittel bei einer Temperatur zwischen 50°C und 90°C in Gegenwart von Mineralsalzen mit einem Amin der allgemeinen Formel III zur Bildung einer Verbindung der allgemeinen Formel I'' kondensiert und anschließend gewünschtenfalls in die optischen Isomeren aufspaltet oder mit einer pharmzeutisch annehmbaren Säure in ein Additionssalz umwandelt.

**Claims** for the Contracting States: BE, CH, DE, FR, GB, IT, LI, LU, NL, SE

1. Compounds of the general formula I:

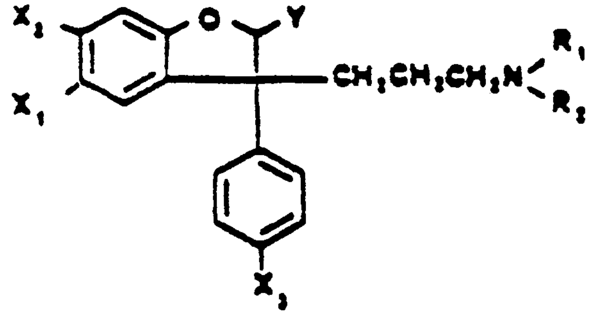

(I)

in which:

- each of $X_1$ and $X_2$, which are identical or different, represents a hydrogen or halogen atom or an alkoxy radical containing from 1 to 4 carbon atoms, or they together form a methylenedioxy radical,
- $X_3$ represents a hydrogen atom or an alkyl radical having from 1 to 4 carbon atoms,
- Y represents a hydrogen atom or a hydroxy radical,
- each of $R_1$ and $R_2$, which are identical or different, represents an alkyl radical having from 1 to 4 carbon atoms, provided that, when Y represents a hydrogen atom, $X_1$ or $X_2$, or $X_1$ and $X_2$ simultaneously, represent(s) an alkoxy radical containing from 1 to 4 carbon atoms,
- or

$R_1$ is a methyl radical and

$R_2$ represents a phenylalkyl group containing from 7 to 9 carbon atoms, or a 2-indanyl group, each of which is optionally substituted on the aromatic ring by one or two alkoxy radicals containing from 1 to 4 carbon atoms, provided that $R_2$ never represents a phenethyl radical when each of $X_1$ and $X_2$ represents a hydrogen or halogen atom and Y represents a hydrogen atom,

- or

$R_1$ and $R_2$ together form, with the nitrogen atom to which they are attached, a 4-morpholinyl radical, a 1,2,6,7-tetrahydrothieno[2,3-c]pyridin-1-yl radical, a tetrahydroisoquinol-2-yl radical optionally substituted by one or two alkoxy radicals having from 1 to 4 carbon atoms, a 4-($C_1$-$C_4$)-alkylpiperazin-1-yl group, or a 4-phenylpiperazin-1-yl group optionally substituted on the aromatic ring by one or two alkoxy radicals containing from 1 to 4 carbon atoms,

in racemic form or in the form of optical isomers, and their addition salts with pharmaceutically acceptable mineral or organic acids.

2. 5-methoxy-3-phenyl-3-[3-(N-(3,4-dimethoxyphenethyl)-N-methylamino)-propyl]-2,3-dihydrobenzofuran, a compound according to the general formula I of claim 1, its optical isomers and its salts with pharmaceutically compatible acids.

3. 3-phenyl-3-[3-(N-3,4-dimethoxyphenethyl)-N-methyl-amino)-propyl]-2,3-dihydrobenzofuran, a compound according to the general formula I of claim 1, its optical isomers and its salts with pharmaceutically compatible acids.

4. Process for the preparation of the compounds of the general formula I of claim 1, characterised in that a 3-aryl-3-(3-chloropropyl)-2-benzofuran of the general formula II:

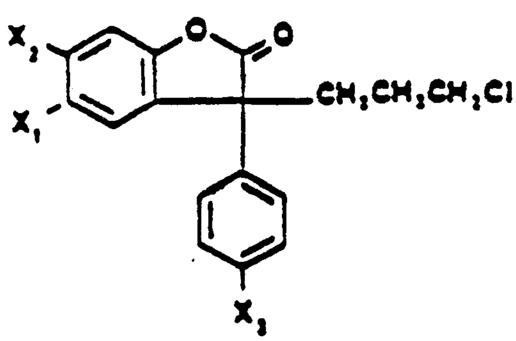

(II)

in which the definition of the substituents $X_1$, $X_2$ and $X_3$ is the same as that given for the general formula I of claim 1, is subjected

either: to the action of a secondary amine of the general formula III:

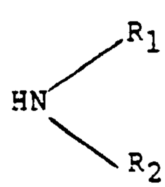

(III)

in which the definition of $R_1$ and $R_2$ is the same as that given for the general formula I of claim 1, in a polar organic solvent at a temperature of between 50°C and 90°C and in the presence of mineral salts, to form a derivative of the general formula IV:

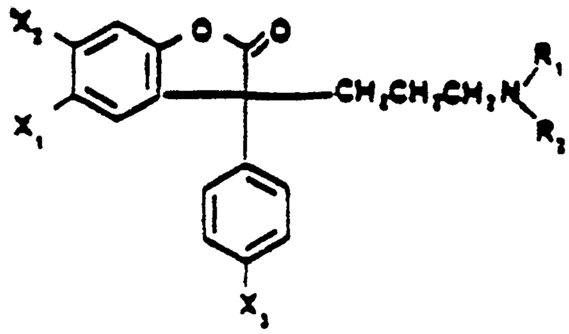

(IV)

in which $X_1$, $X_2$, $X_3$, $R_1$ and $R_2$ have the meanings defined above for the general formula I of claim 1,

- which is partially reduced in an aprotic organic solvent at a temperature of between -50°C and -80°C and in the presence of diisobutylaluminium hydride to form a derivative of the general formula I':

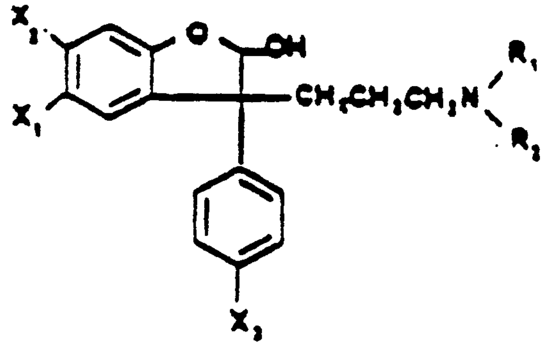

(I')

in which $X_1$, $X_2$, $X_3$, $R_1$ and $R_2$ are as defined above for the general formula I of claim 1,

which is then separated, if desired, into its optical isomers, or is converted into an addition salt with a pharmaceutically acceptable acid,

- or is opened by reduction with the aid of metal hydrides in an inert organic solvent at a temperature of between 15°C and 40°C and is then hydrolysed to form a diol of the general formula V:

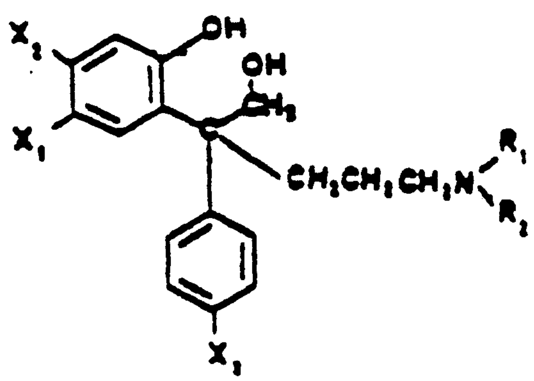

(V)

in which $X_1$, $X_2$, $X_3$, $R_1$ and $R_2$ have the meanings defined above for the general formula I of claim 1, which is cyclised by the action of butyllithium and paratoluenesulphonyl chloride to form a compound of the general formula I'':

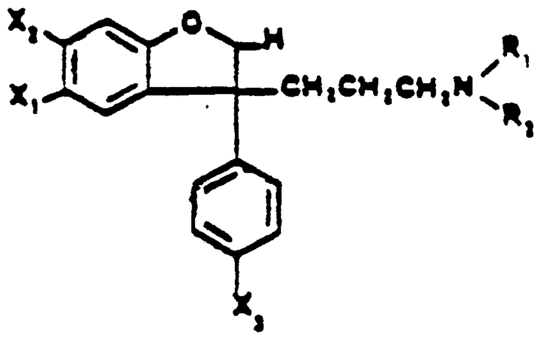

(I")

in which the meanings of $X_1$, $X_2$, $X_3$, $R_1$ and $R_2$ are the same as given for the general formula I of claim 1, which is then separated, if desired, into its optical isomers or salified with a pharmaceutically acceptable acid,

or to the action of a metal hydride in an organic solvent and at a temperature of between 15°C and 40°C in order to obtain, after hydrolysis, a diol of the general formula VI:

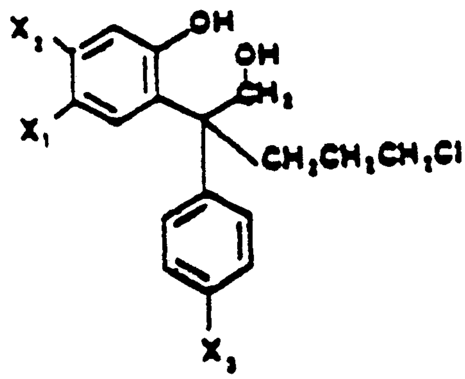

(VI)

in which the definition of $X_1$, $X_2$ and $X_3$ is the same as that given above in the general formula I of claim 1,

which is cyclised by the action of butyllithium and paratoluenesulphonyl chloride in order to obtain a compound of the general formula VII:

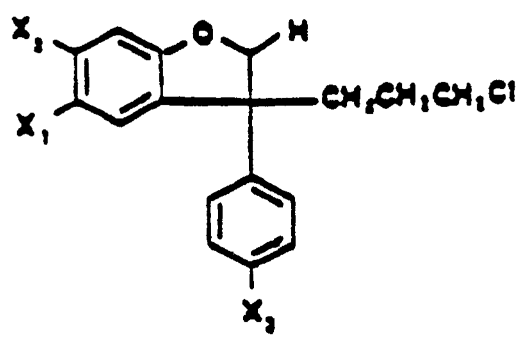

(VII)

in which the definition of $X_1$, $X_2$ and $X_3$ has the meanings defined above for the general formula I of claim 1,

and which is condensed in a polar solvent at a temperature of between 50°C and 90°C and in the presence of mineral salts with an amine of the general formula III in order to obtain a compound of the general formula I"

and, if desired, the optical isomers thereof are then separated or the compound is converted into an addition salt with a pharmaceutically acceptable acid.

5. Pharmaceutical compositions containing as active ingredient a compound according to any one of claims 1 to 3, in association or in admixture with one or more excipients or a pharmaceutically acceptable non-toxic inert carrier.

6. A pharmaceutical composition according to claim 5 that can be used in the treatment of disorders requiring modulators of transmembrane and intracellular movements of calcium.

**Claims** for the Contracting State: AT

1. Process for the preparation of derivatives of the general formula I:

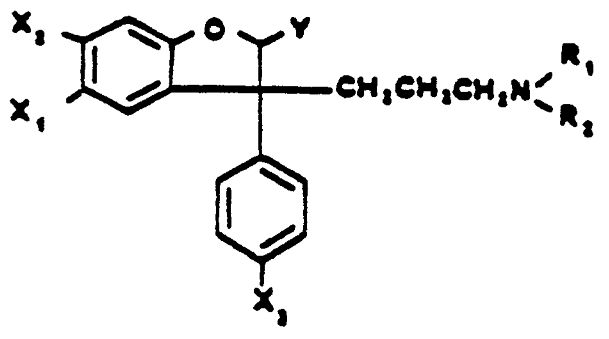

(I)

in which:

- each of $X_1$ and $X_2$, which are identical or different, represents a hydrogen or halogen atom or an alkoxy radical containing from 1 to 4 carbon atoms, or they together form a methylenedioxy radical,
- $X_3$ represents a hydrogen atom or an alkyl radical having from 1 to 4 carbon atoms,
- Y represents a hydrogen atom or a hydroxy radical,
- each of $R_1$ and $R_2$, which are identical or different, represents an alkyl radical having from 1 to 4 carbon atoms, provided that, when Y represents a hydrogen atom, $X_1$ or $X_2$, or $X_1$ and $X_2$ simultaneously, represent(s) an alkoxy radical containing from 1 to 4 carbon atoms,
- or

$R_1$ is a methyl radical and

$R_2$ represents a phenylalkyl group containing from 7 to 9 carbon atoms, or a 2-indanyl group, each of which is optionally substituted on the aromatic ring by one or two alkoxy radicals containing from 1 to 4 carbon atoms, provided that $R_2$ never represents a phenethyl radical when each of $X_1$ and $X_2$ represents a hydrogen or halogen atom and Y represents a hydrogen atom,

- or

$R_1$ and $R_2$ together form, with the nitrogen atom to which they are attached, a 4-morpholinyl radical, a 1,2,6,7-tetrahydrothieno[2,3-c]pyridin-1-yl radical, a tetrahydroisoquinol-2-yl radical optionally substituted by one or two alkoxy radicals having from 1 to 4 carbon atoms, a 4-($C_1$-$C_4$)-alkylpiperazin-1-yl group, or a 4-phenylpiperazin-1-yl group optionally substituted on the aromatic ring by one or two alkoxy radicals containing from 1 to 4 carbon atoms,

in racemic form or in the form of optical isomers, and their addition salts with pharmaceutically acceptable mineral or organic acids,

characterised in that a 3-aryl-3-(3-chloropropyl)-2-benzofuran of the general formula II:

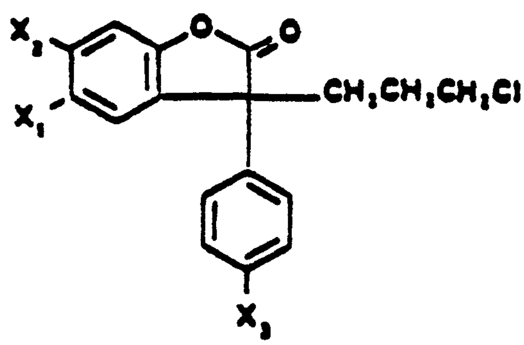

(II)

in which the definition of the substituents $X_1$, $X_2$ and $X_3$ is the same as that given for the general formula I, is subjected

<u>either</u>: to the action of a secondary amine of the general formula III:

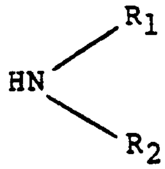

(III)

in which the definition of $R_1$ and $R_2$ is the same as that given above for the general formula I, in a polar organic solvent at a temperature of between 50° C and 90° C and in the presence of mineral salts, to form a derivative of the general formula IV:

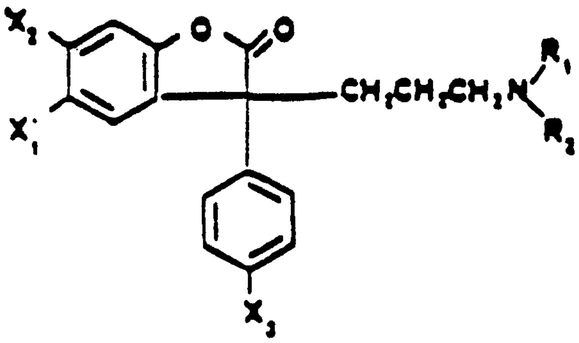

(IV)

in which $X_1$, $X_2$, $X_3$, $R_1$ and $R_2$ have the meanings defined above for the formula I,

- which is partially reduced in an aprotic organic solvent at a temperature of between -50° C and -80° C and in the presence of diisobutylaluminium hydride to form a derivative of the general formula I':

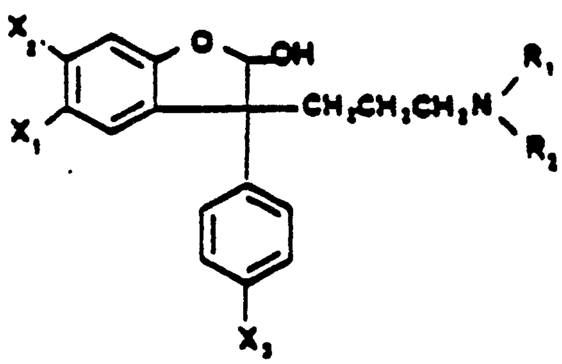

(I')

in which $X_1$, $X_2$, $X_3$, $R_1$ and $R_2$ are as defined above for the general formula I,

which is then separated, if desired, into its optical isomers, or is salified with a pharmaceutically acceptable acid,

- or is opened by reduction with the aid of metal hydrides in an inert organic solvent at a temperature of between 15° C and 40° C and is then hydrolysed to form a diol of the general formula V:

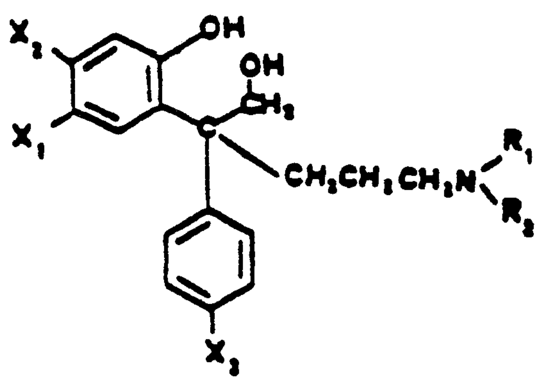

(V)

in which $X_1$, $X_2$, $X_3$, $R_1$ and $R_2$ have the meanings defined above for the general formula I,

which is cyclised by the action of butyllithium and paratoluenesulphonyl chloride to form a compound of the general formula I":

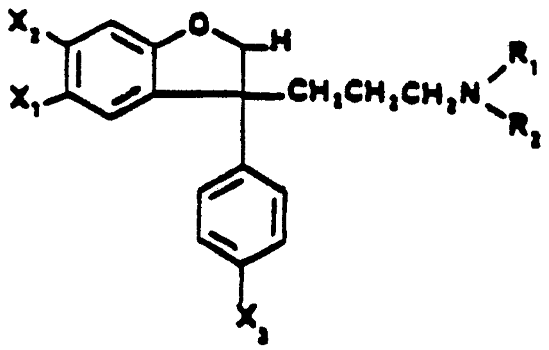

(I")

in which the meanings of $X_1$, $X_2$, $X_3$, $R_1$ and $R_2$ are the same as given for the general formula I,

which is then separated, if desired, into its optical isomers or salified with a pharmaceutically acceptable acid,

or to the action of a metal hydride in an organic solvent and at a temperature of between 15°C and 40°C in order to obtain, after hydrolysis, a diol of the general formula VI:

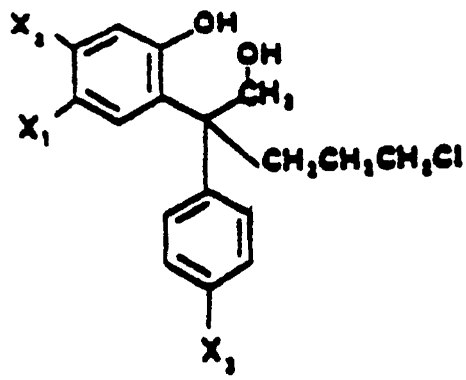

(VI)

in which the definition of $X_1$, $X_2$ and $X_3$ is the same as that given above in the general formula I, which is cyclised by the action of butyllithium and paratoluenesulphonyl chloride in order to obtain a compound of the general formula VII:

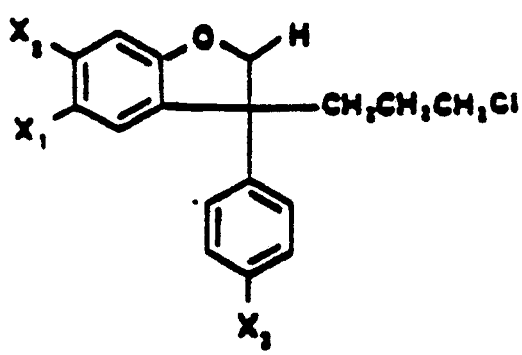

(VII)

in which the definition of $X_1$, $X_2$ and $X_3$ has the meanings defined above for the general formula I, and which is condensed in a polar solvent at a temperature of between 50°C and 90°C and in the presence of mineral salts with an amine of the general formula III in order to obtain a compound of the general formula I"

and, if desired, the optical isomers thereof are then separated or the compound is converted into an addition salt with a pharmaceutically acceptable acid.